# EUROPEAN PATENT APPLICATION

(11) **EP 4 656 630 A1**
(43) Date of publication of application: **03.12.2025**
(21) Application number: 24747473.7
(22) Date of filing: 25.01.2024
(51) Int. Cl.: C07D 277/82, A61K 31/428, A61K 31/437, A61K 31/519, C07D 513/04, C07D 487/04, C07D 417/12, C07D 413/12, C07D 207/16, C07D 498/04

(54) **NOVEL COMPOUND AS LYSYL-TRNA SYNTHETASE 1 INHIBITOR, AND USE THEREOF**

(30) Priority: 25.01.2023 KR 20230009752
(71) Applicant: Zymedi Co., Ltd., Incheon 21893 (KR)
(72) Inventor: KWON, Hyuk Sang, Incheon 21893 (KR); KWON, Nam Hoon, Incheon 21893 (KR); PARK, Choul Hong, Incheon 21893 (KR); OH, Jin Soo, Incheon 21893 (KR); ROH, Chae Woon, Incheon 21893 (KR)
(74) Representative: Schwarz & Partner Patentanwälte GmbH
(86) International application number: PCT/KR2024/001237
(87) International publication number: WO 2024/158240

(57) **Abstract**

The present invention relates to a novel compound as a lysyl-tRNA synthetase 1 (KARS 1) inhibitor, and a use thereof, and, more specifically, to a novel compound as a lysyl-tRNA synthetase 1 inhibitor, and a use of preventing or treating immune cell migration-associated diseases and cancer metastasis.

## Description

### [Technical Field]

The present application claims priority to Korean Patent Application No. 10-2023-0009752, filed on January 25, 2023, and the entire disclosure of the specification is incorporated herein by reference.

The present application relates to novel compounds as lysyl-tRNA synthetase 1 (KARS1) inhibitors and their uses, and more specifically, to novel compounds as lysyl-tRNA synthetase 1 inhibitors and their uses for the prevention or treatment of immune cell migration-related diseases and cancer metastasis.

### [Background of the Invention]

In various tissues of the body, each cell migrates in different ways depending on its genetic characteristics and environment. Uncontrolled cell migration is associated with various disease states such as inflammatory diseases and cancer metastasis, but the signaling and mechanism characteristics of migration specific to each cell have not been fully elucidated. In particular, it has been reported that even the same factor interacts differently depending on the cell type, adding difficulty to elucidating the signaling process and mechanisms. For example, AQP1 (water channel aquaporin-1) is known to promote cell migration in epithelial cells and is particularly known to promote cancer metastasis (Hara-Chikuma M et al., Aquaporin-1 facilitates epithelial cell migration in kidney proximal tubule, J Am Soc Nephrol. 2006 Jan;17(1):39-45; Jiang Y, Aquaporin-1 activity of plasma membrane affects HT20 colon cancer cell migration, IUBMB Life. 2009 Oct;61(10):1001-9). However, in the case of macrophages, despite expressing AQP1, it has been reported that this inhibits macrophage migration (Tyteca D et al., Regulation of Macrophage Motility by the Water Channel Aquaporin-1: Crucial Role of M0/M2 Phenotype Switch, PLoS One. 2015 Feb 26;10(2):e0117398). As such, since various modes and characteristics of migration exist for each cell, drugs designed to prevent the migration of specific cells have shown significant limitations and insufficient efficacy. Therefore, there is a need to explore new strategies to control the migratory switch of cells and treat migration-related diseases.

Meanwhile, immune cells are not only the primary defense mechanism in the body but also have recently been reported to play a major role in the pathogenesis of diseases due to excessive activation. When inflammatory immune cells are activated, an increase in immune cell mobility is generally observed. Specifically, it has been reported that such immune cell migration and infiltration are closely related to the pathology of the following diseases.

For example, cardiovascular diseases are diseases that occur in the heart and major arteries, including atherosclerosis and coronary artery disease. Atherosclerosis is an inflammatory disease caused by cholesterol and develops due to plaques composed of cholesterol deposited on the inner lining of arteries and immune cells that have migrated from the blood into the arterial wall. That is, cholesterol oxidation products cause inflammation, and immune cells such as monocytes migrate to the inflamed area, forming plaques. When plaques form, the inner surface of the blood vessel becomes rough, the walls thicken, and the diameter of the inner blood flow narrows, causing circulatory disorders. If the fibrous cap surrounding the plaque ruptures, thrombosis occurs inside the blood vessel, and bleeding into the plaque causes the vascular lumen to narrow or become blocked, leading to cardiovascular diseases. It has been newly suggested that CCL2 (CC-Chemokine ligand 2, MCP-1), which induces monocyte migration and causes inflammatory responses, plays a crucial role in the development and progression of such cardiovascular diseases. By inhibiting the action of CCL2 and the subsequent migration of monocytes, methods for treating such cardiovascular diseases have been proposed (Gu L et al., Mol Cell, 1998;2(2):275-81, Aiello RJ et al., Arterioscler Thromb Vasc Biol 1999;19(6):1518-25, Gosling J1 et al., Clin Invest 1999;103(6):773-8, Harrington JR et al., Stem Cells 2000;18(1):65-6, Ikeda U et al., Clin Cardiol 2002;25(4):143-7). Additionally, in hypertension, various immune cells that secrete inflammatory cytokines excessively migrate to blood vessels, leading to thickening of the vascular walls and loss of vascular elasticity.

Furthermore, in pulmonary arterial hypertension (PAH), although various predisposing factors such as genetics, infections, and associated diseases are involved, immune responses due to endothelial cell injury are known to act as key pathological factors (Huertas et al., Circulation, 129:1332-1340, 2014). This phenomenon is known to be deeply associated with a series of processes caused by immune cell infiltration and dysfunction, and particularly, the interaction between immune cells and vascular endothelial cells is known to be important in PAH. Moreover, recent reports have indicated that in Alport syndrome, the infiltration of monocytes and macrophages accelerates the progression of the disease.

Meanwhile, in fibrosis-related diseases, persistent (chronic) inflammatory responses activate the wound-healing program, which leads to fibrosis. After tissue damage, inflammatory immune cells such as monocytes/macrophages, neutrophils, eosinophils, and mast cells rapidly infiltrate the damaged area, become activated, and secrete various cytokines. These cytokines activate surrounding fibroblasts, epithelial cells, and smooth muscle cells into myoblast-like cells. These myoblast-like cells produce and secrete large amounts of extracellular matrix proteins, ultimately causing excessive accumulation of extracellular matrix proteins in the tissue, leaving scars and inducing tissue fibrosis or hypertrophy (Gurtner GC et al., Trends Cell Biol. 15:599-607, 2005). This pathological mechanism is one of the fundamental causes of scar formation in skin tissue due to wounds, burns, and pressure sores, as well as sclerotic fibrosis in tissues such as the liver, kidneys, blood vessels, and lungs. Additionally, fibrosis is a major pathological characteristic in chronic autoimmune diseases such as scleroderma, rheumatoid arthritis, Crohn's disease, ulcerative colitis, myelofibrosis, and systemic lupus erythematosus. Furthermore, the activation of inflammatory immune cells is known to contribute to pathological phenomena in atopic diseases, asthma, COPD, psoriasis, keloids, and proliferative retinopathy.

In particular, in the wound-healing program, fibroblasts activated into myoblast-like cells are called myofibroblasts. Since myofibroblasts are central to the pathology of all fibrosis-related diseases, eliminating the molecular biological or immunological mechanisms that induce the activation of myofibroblasts becomes a key factor in disease treatment. However, since the removal of inflammatory responses is not easily achieved in practice, understanding the mechanisms of innate and adaptive immunity to identify key mediators is important for slowing fibrosis.

Monocytes and macrophages contribute to wound healing but also secrete reactive oxygen/nitrogen species, which adversely affect surrounding cells. Therefore, limiting monocytes and macrophages, which are the first to respond in the early stages of the disease, is considered a therapeutic strategy for various chronic inflammatory and fibrosis-related diseases.

When the wound-healing mechanism triggers a fibrotic response, PDGF (platelet-derived growth factor), which is related to blood cell aggregation, is known to recruit other inflammatory immune cells to the wound site, and TGF-β1 is known to promote extracellular matrix synthesis from local fibroblasts. However, it has been reported that even in cases where these factors related to blood cell aggregation are deficient, fibrosis is induced.

As described above, in diseases where excessive immune cell activation is problematic, target factors for blocking the migration (and infiltration) of immune cells have been proposed, and attempts have been made to devise therapeutic methods targeting these factors. However, limitations of each approach have been reported. Accordingly, identifying the key mediators in immune cell migration and finding strategies to control them remain important challenges for effective disease treatment.

### [Problem to be Solved]

Accordingly, the inventors of the present invention, while conducting research to find a new therapeutic strategy for immune cell migration (infiltration)-related diseases through existing studies, confirmed that the phenomenon of increased KARS1 levels in the cell membrane region of immune cells (monocytes/macrophages) is an important pathological phenomenon related to immune cell migration and infiltration-related diseases. Furthermore, they confirmed that novel compounds represented by the general formula of Formula 1 inhibit the activity of KARS1, thereby suppressing immune cell migration and infiltration, inhibiting the migration of cancer cells, and effectively treating related diseases, thus completing the present invention.

Therefore, an object of the present invention is to provide a compound of Formula 1, an isomer thereof, or a pharmaceutically acceptable salt thereof: 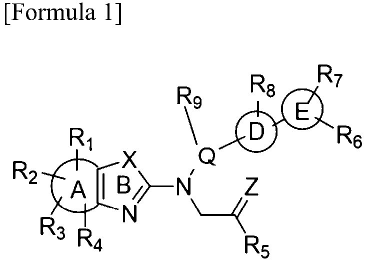
X is O, NH, S, or CH=N,
A is C5-C10 aryl forming a bicyclic aromatic ring or 5- to 10-membered heteroaryl containing one or more heteroatoms selected from the group consisting of N, O, and S, together with B
R₁, R₂, R₃, R₄ are each independently absent, hydrogen, halogen, =O, C1-C6 alkyl, or amine,
Z is two H or O,
R₅ is C5-C10 aryl or pyridinonyl substituted or unsubstituted with C1-C6 alkoxy, -(C=O)NH₂, or -OCF₃,
Q is hydrogen or methylene,
D is carboxyl, C5-C10 aryl, benzoimidazolyl, benzoxazolyl, isoindolinyl, -C=O-, -C₆H₄C=O-, or -C₆H₄NH-,
R₈ is hydrogen or C1-C6 alkyl,
E is, when D is aryl, phenyl, C1-C6 alkylene, C2-C8 alkylaminocarbonyl, 3- to 10-membered heterocycloalkyl containing one or more heteroatoms selected from the group consisting of N, O, and S, 5- to 10-membered heteroaryl containing one or more heteroatoms selected from the group consisting of N, O, and S, C3-C10 cycloalkylamine, C3-C10 cycloalkyl, -B(OH)O-, - CH₂CH₂(C=O), -C=C-, -C=C-(C=O)N-, -cyclobutyl-(C=O)-, -azetinyl-(C=O)-, -C₆H₄-(C=O)-, - pyrrolidinyl-(C=O)-, -furanyl-(C=O)-, -piperidinyl-(C=O)-, -pyrazolyl-(C=O)-, or pyrrolyl-(C=O)-, wherein R₆, R₇ are each independently absent, hydrogen, carboxylic acid, C1-C6 alkyl, C2-C8 alkylaminocarbonyl, aminocarbonyl, hydroxy, or -Si(CH₃)₃,
E is, when A is pyrrole or pyridone and D is aryl, hydrogen, halogen, -C≡C-, -C≡C-(C=O), wherein R6, R7 are each independently absent, hydrogen, carboxyl, OH, CH3, CH2CH3, -Si(CH3)3,
E is, when D is -C=O-, benzoimidazolyl, benzoxazolyl, isoindolinyl, -C₆H₄C=O-, or -C₆H₄NH-, absent, -B(OH)₂, carboxyl, hydrogen, aminocarbonyl, aminosulfonyl, C1-C6 alkylene, -(C=O)-, - C=C-, -S(=O)₂-, -CH₂(C=O)-, -B(OH)O-, -CHR₁₀(C=O)-, -NHCHR₁₀(C=O)-, cyclobutyl-(C=O)-, furanyl-(C=O)-, or pyrrolidinyl-(C=O)-, wherein R₆, R₇ are each independently absent, carboxyl, hydrogen, hydroxy, C1-C6 alkyl, or amine,
R₉ is hydrogen or C1-C6 alkyl,
R₁₀ is hydrogen, C1-C6 alkyl, or C1-C6 hydroxyalkyl.

Another object of the present invention is to provide a pharmaceutical composition for preventing or treating immune cell migration-related diseases comprising the compound, an isomer thereof, or a pharmaceutically acceptable salt thereof as an active ingredient.

Another object of the present invention is to provide a pharmaceutical composition for preventing or inhibiting cancer metastasis comprising the compound, an isomer thereof, or a pharmaceutically acceptable salt thereof as an active ingredient.

Another object of the present invention is to provide the use of the compound, an isomer thereof, or a pharmaceutically acceptable salt thereof for manufacturing a composition for treating immune cell migration-related diseases.

Another object of the present invention is to provide a method for treating immune cell migration-related diseases comprising administering an effective amount of a composition comprising the compound, an isomer thereof, or a pharmaceutically acceptable salt thereof as an active ingredient to a subject in need thereof.

Another object of the present invention is to provide the use of the compound, an isomer thereof, or a pharmaceutically acceptable salt thereof for manufacturing a composition for inhibiting cancer metastasis.

Another object of the present invention is to provide a method for inhibiting cancer comprising administering an effective amount of a composition comprising the compound, an isomer thereof, or a pharmaceutically acceptable salt thereof as an active ingredient to a subject in need thereof.

### [Means for Solving the Problem]

To achieve the aforementioned objectives of the present invention, the present invention provides a compound of the following Formula 1, an isomer thereof, or a pharmaceutically acceptable salt thereof: 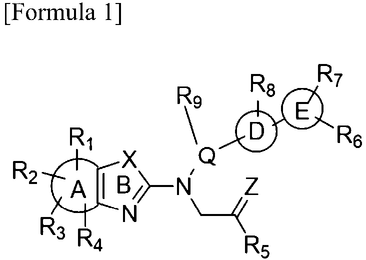
X is O, NH, S, or CH=N,
A is C5-C10 aryl forming a bicyclic aromatic ring or 5- to 10-membered heteroaryl containing one or more heteroatoms selected from the group consisting of N, O, and S, together with B
R₁, R₂, R₃, R₄ are each independently absent, hydrogen, halogen, =O, C1-C6 alkyl, or amine,
Z is two H or O,
R₅ is C5-C10 aryl or pyridinonyl substituted or unsubstituted with C1-C6 alkoxy, -(C=O)NH₂, or -OCF₃,
Q is hydrogen or methylene,
D is carboxyl, C5-C10 aryl, benzoimidazolyl, benzoxazolyl, isoindolinyl, -C=O-, -C₆H₄C=O-, or -C₆H₄NH-,
R8 is hydrogen or C1-C6 alkyl,
E is, when D is aryl, phenyl, C1-C6 alkylene, C2-C8 alkylaminocarbonyl, 3- to 10-membered heterocycloalkyl containing one or more heteroatoms selected from the group consisting of N, O, and S, 5- to 10-membered heteroaryl containing one or more heteroatoms selected from the group consisting of N, O, and S, C3-C10 cycloalkylamine, C3-C10 cycloalkyl, -B(OH)O-, - CH₂CH₂(C=O), -C≡C-, -C≡C-(C=O)N-, -cyclobutyl-(C=O)-, -azetinyl-(C=O)-, -C₆H₄-(C=O)-, - pyrrolidinyl-(C=O)-, -furanyl-(C=O)-, -piperidinyl-(C=O)-, -pyrazolyl-(C=O)-, or pyrrolyl-(C=O)-, wherein R₆, R₇ are each independently absent, hydrogen, carboxylic acid, C1-C6 alkyl, C2-C8 alkylaminocarbonyl, aminocarbonyl, hydroxy, or -Si(CH₃)₃,
E is, when A is pyrrole or pyridone and D is aryl, hydrogen, halogen, -C≡C-, -C≡C-(C=O), wherein R₆, R₇ are each independently absent, hydrogen, carboxyl, OH, CH₃, CH₂CH₃, -Si(CH₃)₃,
E is, when D is -C=O-, benzoimidazolyl, benzoxazolyl, isoindolinyl, -C₆H₄C=O-, or -C₆H₄NH-, absent, -B(OH)₂, carboxyl, hydrogen, aminocarbonyl, aminosulfonyl, C1-C6 alkylene, -(C=O)-, - C=C-, -S(=O)₂-, -CH₂(C=O)-, -B(OH)O-, -CHR₁₀(C=O)-, -NHCHR₁₀(C=O)-, cyclobutyl-(C=O)-, furanyl-(C=O)-, or pyrrolidinyl-(C=O)-, wherein R₆, R₇ are each independently absent, carboxyl, hydrogen, hydroxy, C1-C6 alkyl, or amine,
R₉ is hydrogen or C1-C6 alkyl,
R₁₀ is hydrogen, C1-C6 alkyl, or C1-C6 hydroxyalkyl.

To achieve another objective of the present invention, the present invention provides a pharmaceutical composition for preventing or treating immune cell migration-related diseases comprising the compound, an isomer thereof, or a pharmaceutically acceptable salt thereof as an active ingredient.

To achieve another objective of the present invention, the present invention provides a pharmaceutical composition for preventing or inhibiting cancer metastasis comprising the compound, an isomer thereof, or a pharmaceutically acceptable salt thereof as an active ingredient.

To achieve another objective of the present invention, the present invention provides the use of the compound, an isomer thereof, or a pharmaceutically acceptable salt thereof for manufacturing a composition for treating immune cell migration-related diseases.

To achieve another objective of the present invention, the present invention provides a method for treating immune cell migration-related diseases comprising administering an effective amount of a composition comprising the compound, an isomer thereof, or a pharmaceutically acceptable salt thereof as an active ingredient to a subject in need thereof.

To achieve another objective of the present invention, the present invention provides the use of the compound, an isomer thereof, or a pharmaceutically acceptable salt thereof for manufacturing a composition for inhibiting cancer metastasis.

To achieve another objective of the present invention, the present invention provides a method for inhibiting cancer comprising administering an effective amount of a composition comprising the compound, an isomer thereof, or a pharmaceutically acceptable salt thereof as an active ingredient to a subject in need thereof.

In the present specification, unless otherwise specified, the alkyl, alkenyl, or alkynyl substituents or alkynyl alkenyl portions of substituents may be linear or branched (branched chain, side chain). Alkyl and alkenyl chains may also include intervening heteroatoms such as oxygen.

Cx-Cy alkyl refers to a saturated aliphatic hydrocarbon group having x-y carbon atoms, which may be linear or branched. C1-C6 alkyl contains 1 to 6 carbon atoms. "Branched" means that one or more carbon branching points are present in the group. For example, tert-butyl and isopropyl are both branched groups. Examples of C1-C6 alkyl groups include methyl, ethyl, propyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, tert-butyl, n-pentyl, isopentyl, neopentyl, and n-hexyl.

Cx-Cy alkylene residues refer to divalent hydrocarbon groups having one less hydrogen atom than the Cx-Cy alkyl defined above, which may be linear or branched. Non-limiting examples of C1-C6 alkylene groups include methylene, ethylene, n-propylene, n-butylene, methylmethylene, and dimethylmethylene.

C2-C6 alkenyl refers to a linear or branched hydrocarbon chain radical containing at least 2 carbon atoms and one or more double bonds. Non-limiting examples of alkenyl groups include ethenyl, propenyl, 2-propenyl, 1-butenyl, 2-butenyl, 1-hexenyl, 2-methyl-1-propenyl, 1,2-butadienyl, 1,3-pentadienyl, 1,4-pentadienyl, and 1-hexadienyl.

C1-C6 alkoxy refers to a group or part of a group having an -O-Cx-Cy alkyl group as defined above. Non-limiting examples of C1-C6 alkoxy include methoxy, ethoxy, propoxy, isopropoxy, butoxy, pentyloxy, and hexyloxy.

Halogen or halo refers to a chlorine (Cl), bromine (Br), fluorine (F), or iodine (I) atom.

Cx-Cy cycloalkyl refers to a cyclic non-aromatic hydrocarbon group having x-y carbon atoms. C3-C10 cycloalkyl refers to a hydrocarbon ring containing 3-10 carbon atoms. Non-limiting examples of C3-C10 cycloalkyl include cyclopropyl, cyclobutyl, cyclopentyl, cyclohexyl, cyclopentyl, cyclohexyl, cycloheptyl, cyclooctyl, cyclononyl, and cyclodecyl.

For examples of aryl groups having 12 or fewer carbon atom ring members, it refers to any monocyclic or bicyclic hydrocarbon group containing at least one aromatic group. Non-limiting examples of aryl groups include phenyl, naphthyl, tetrahydronaphthyl, and biphenyl.

Heteroaryl groups may be monocyclic or bicyclic. Bicyclic rings may be fused aromatic rings, where the rings may be aromatic or a non-aromatic ring may be fused to an aromatic ring. Heteroaryl includes 1, 2, or 3 heteroatoms selected from oxygen (O), sulfur (S), and nitrogen (N). If the heteroatom is nitrogen, it may be oxidized. Non-limiting examples of heteroaryl include pyridinyl, pyrazinyl, pyrimidinyl, pyridazinyl, furyl, thiophenyl, pyrrolyl, oxazolyl, thiazolyl, pyrazolyl, triazolyl, tetrazolyl, indolyl, indolizinyl, isoindolyl, indolinyl, purinyl, pyrazinyl, imidazolyl, indazolyl, isothiazolyl, isoxazolyl, oxadiazolyl, oxazinyl, tetrazolyl, thiadiazolyl, benzofuranyl, isobenzofuranyl, benzothiophenyl, isobenzothiophenyl, benzimidazolyl, benzothiazolyl, naphthyridinyl, piperidinyl, pyrazinyl, 4H-quinolizinyl, quinolinyl, isoquinolinyl, cinnolinyl, phthalazinyl, quinazolinyl, imidazopyridinyl, pyrazolopyridinyl, thiazolopyridinyl, indolinyl, isoindolinyl, triazinyl, pyridazinyl, and quinoxalinyl.

The heterocyclyl group may also be a single ring or may include two or more fused rings that are saturated or partially unsaturated and contain 1, 2, or 3 heteroatoms selected from oxygen (O), sulfur (S), and nitrogen (N). Non-limiting examples of heterocyclyl include azetidinyl, pyrrolidinyl, piperidinyl, azepanyl, diazepanyl, dihydrofuran (i.e., 2,3-dihydrofuran, 2,5-dihydrofuran), 4,5-dihydro-1H-maleimido, dioxolanyl, morpholinyl, oxazolidinyl, piperazinyl, tetrahydrofuran, thiomorpholinyl, dihydropyranyl (i.e., 3,4-dihydropyranyl, 3,6-dihydropyranyl), dioxanyl, hexahydropyrimidinyl, pyrazolinyl, pyrazolidinyl, pyridazinyl, 4H-quinolizinyl, quinuclidinyl, tetrahydropyranyl, tetrahydropyridinyl, tetrahydropyrimidinyl, tetrahydrothiophenyl, tetramethylenesulfoxide, thiazolidinyl, hydantoinyl, benzopyranyl, tetrahydrothiazolopyridinyl, tetrahydroquinolinyl, tetrahydroisoquinolinyl, tetrahydropyrazolopyrazinyl, and tetrahydrothiazoloazepinyl.

The term "pharmaceutically acceptable" refers to compositions and molecular entities that are physiologically acceptable and do not generally cause allergic reactions or similar adverse reactions, such as gastrointestinal disturbances or dizziness, when administered to humans or animals. For example, the term "pharmaceutically acceptable" means that the molecule, etc., has been approved by state or federal regulatory agencies in the United States or is included in the United States Pharmacopeia or other generally recognized pharmacopeia for use in animals, particularly humans.

General methods for the preparation of salts are well known to those skilled in the art. The salts may be formed by conventional means through the reaction of the free acid or free base form of the compound with one or more equivalents of a suitable counterpart, optionally in a solvent. The salts may be insoluble, and the solvent or medium may then be removed using standard techniques (e.g., vacuum, freeze-drying, or filtration). Salts may also be prepared by exchanging the counterion of the compound with another counterion in the form of a salt using an aqueous ion exchange resin.

Pharmaceutically acceptable salts, hydrates, solvates, tautomers, polymorphs, and prodrugs of the compounds described herein are also provided. The term "pharmaceutically acceptable" or "physiologically acceptable" refers to compounds, salts, compositions, formulations, and other substances that are suitable for veterinary or human pharmaceutical use in the preparation of pharmaceutical compositions.

The compounds described herein may be prepared and/or formulated as pharmaceutically acceptable salts or, where appropriate, as free bases. Pharmaceutically acceptable salts are non-toxic salts of the free base form of the compound that retain the desired pharmacological activity of the free base. These salts may be derived from inorganic or organic acids or bases. For example, compounds containing basic nitrogen may be prepared as pharmaceutically acceptable salts by contacting the compound with an inorganic or organic acid. Non-limiting examples of pharmaceutically acceptable salts include sulfates, pyrosulfates, bisulfates, sulfites, bisulfites, phosphates, monohydrogen phosphates, dihydrogen phosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, methylsulfonates, propylsulfonates, besylates, xylenesulfonates, naphthalene-1-sulfonates, naphthalene-2-sulfonates, phenylacetates, phenylpropionates, phenylbutyrates, citrates, lactates, γ-hydroxybutyrates, glycolates, tartrates, and mandelates. Other suitable lists of pharmaceutically acceptable salts are known to those skilled in the art.

Examples of "pharmaceutically acceptable salts" of the compounds disclosed herein also include salts derived from suitable bases such as alkali metals (e.g., sodium, potassium), alkaline earth metals (e.g., magnesium), ammonium, and N(C1-C4 alkyl)4+. Base addition salts such as sodium or potassium salts are also included.

General methods for the preparation of salts are well known to those skilled in the art. The salts may be formed by conventional means through the reaction of the free acid or free base form of the compound with one or more equivalents of a suitable counterpart, optionally in a solvent. The salts may be insoluble, and the solvent or medium may then be removed using standard techniques (e.g., vacuum, freeze-drying, or filtration). Salts may also be prepared by exchanging the counterion of the compound with another counterion in the form of a salt using an aqueous ion exchange resin.

Additionally, the compounds described herein, or their pharmaceutically acceptable salts, isomers, or mixtures thereof, in which 1 to n hydrogen atoms attached to a carbon atom are replaced with deuterium atoms or D, are provided, where n is the number of hydrogen atoms in the molecule. As is known in the art, deuterium is a non-radioactive isotope of hydrogen. Such compounds may increase metabolic resistance and may be useful in increasing the half-life of the compounds described herein or their pharmaceutically acceptable salts, isomers, or mixtures thereof when administered to mammals.

Examples of isotopes that may be incorporated into the disclosed compounds include isotopes of hydrogen, carbon, nitrogen, oxygen, phosphorus, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I. Substitution with positron-emitting isotopes such as ¹¹C, ¹⁸F, ¹⁵O, and ¹³N may be useful in positron emission tomography (PET) studies to investigate substrate receptor occupancy. Isotope-labeled compounds of Formula (I) may generally be prepared by conventional techniques known to those skilled in the art or by methods similar to those described in the examples below, using appropriate isotope-labeled reagents in place of previously used unlabeled reagents.

The compounds of the embodiments disclosed herein or their pharmaceutically acceptable salts may include one or more asymmetric centers and may form enantiomers, diastereomers, and other stereoisomers that can be defined in terms of absolute stereochemistry as (R)- or (S)-, or in the case of amino acids, as (D)- or (L)-. The present disclosure is intended to include all such possible isomers as well as their racemic and optically pure forms. Optically active (+) and (-), (R)- and (S)-, or (D)- and (L)- isomers may be prepared using chiral synthons or chiral reagents or may be separated using conventional techniques such as chromatography and fractional crystallization. Conventional techniques for the preparation/separation of individual enantiomers include chiral synthesis from appropriate optically pure precursors or the resolution of racemates (or racemates of salts or derivatives) using, for example, chiral high-performance liquid chromatography (HPLC). Where the compounds described herein include olefinic double bonds or other geometrically asymmetric centers, and unless otherwise specified, the compounds are intended to include both E and Z geometric isomers. Similarly, all tautomers are also intended to be included. Where a compound is represented in its chiral form, the embodiments are understood to include, but are not limited to, forms enriched in specific diastereomers or enantiomers. Where chirality is not specified but is present, the embodiments are understood to relate to forms enriched in specific diastereomers or enantiomers; or to racemic or scalemic mixtures of such compounds. As used herein, a "scalemic mixture" is a mixture of stereoisomers in a ratio other than 1:1.

Stereoisomers are compounds in which the same atoms are bonded by the same bonds but have different three-dimensional structures and are non-interchangeable. The present disclosure contemplates various stereoisomers and mixtures thereof, including "enantiomers," which refer to two stereoisomers that are non-superimposable mirror images of each other.

Tautomers refer to the transfer of a proton from one atom in a molecule to another atom in the same molecule. In some embodiments, the present disclosure includes tautomers of the compounds described herein.

Solvates refer to the result of the interaction between a solvent and a compound. Solvates of the salts of the compounds described herein are also provided. Hydrates of the compounds described herein are also provided.

Hydrates refer to the compounds of the present disclosure chemically bound to one or more water molecules.

Hereinafter, the present invention will be described in detail.

The present invention provides a compound of Chemical Formula 1, an isomer thereof, or a pharmaceutically acceptable salt thereof: 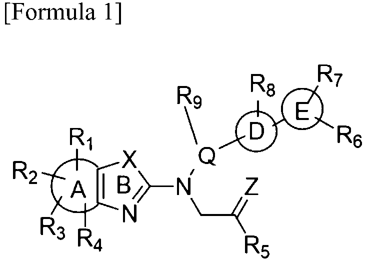 wherein
X is O, NH, S, or CH=N,
A is C5-C10 aryl forming a bicyclic aromatic ring or 5- to 10-membered heteroaryl containing one or more heteroatoms selected from the group consisting of N, O, and S together with B,
R₁, R₂, R₃, R₄ are each independently absent, hydrogen, halogen, =O, C1-C6 alkyl, or amine,
Z is two H or O,
R₅ is C5-C10 aryl or pyridinonyl substituted or unsubstituted with C1-C6 alkoxy, -(C=O)NH₂, or -OCF₃,
Q is hydrogen or methylene,
D is carboxyl, C5-C10 aryl, benzoimidazolyl, benzoxazolyl, isoindolinyl, -C=O-, -C₆H₄C=O-, or -C₆H₄NH-,
R₈ is hydrogen or C1-C6 alkyl,
E is, when D is aryl, phenyl, C1-C6 alkylene, C2-C8 alkylaminocarbonyl, 3- to 10-membered heterocycloalkyl containing one or more heteroatoms selected from the group consisting of N, O, and S, 5- to 10-membered heteroaryl containing one or more heteroatoms selected from the group consisting of N, O, and S, C3-C10 cycloalkylamine, C3-C10 cycloalkyl, -B(OH)O-, - CH₂CH₂(C=O), -C=C-, -C=C-(C=O)N-, -cyclobutyl-(C=O)-, -azetinyl-(C=O)-, -C₆H₄-(C=O)-, - pyrrolidinyl-(C=O)-, -furanyl-(C=O)-, -piperidinyl-(C=O)-, -pyrazolyl-(C=O)-, or pyrrolyl-(C=O)-, wherein R₆, R₇ are each independently absent, hydrogen, carboxylic acid, C1-C6 alkyl, C2-C8 alkylaminocarbonyl, aminocarbonyl, hydroxy, or -Si(CH₃)₃,
E is, when A is pyrrole or pyridone and D is aryl, hydrogen, halogen, -C≡C-, -C=C-(C=O), wherein R₆, R₇ are each independently absent, hydrogen, carboxyl, OH, CH₃, CH₂CH₃, -Si(CH₃)₃,
E is, when D is -C=O-, benzoimidazolyl, benzoxazolyl, isoindolinyl, -C₆H₄C=O-, or -C₆H₄NH-, absent, -B(OH)₂, carboxyl, hydrogen, aminocarbonyl, aminosulfonyl, C1-C6 alkylene, -(C=O)-, - C≡C-, -S(=O)₂-, -CH₂(C=O)-, -B(OH)O-, -CHR₁₀(C=O)-, -NHCHR₁₀(C=O)-, cyclobutyl-(C=O)-, furanyl-(C=O)-, or pyrrolidinyl-(C=O)-, wherein R₆, R₇ are each independently absent, carboxyl, hydrogen, hydroxy, C1-C6 alkyl, or amine,
R₉ is hydrogen or C1-C6 alkyl,
R₁₀ is hydrogen, C1-C6 alkyl, or C1-C6 hydroxyalkyl.

In one aspect of the present invention, A may be benzene, pyridine, pyrimidine, pyridone, or pyrrole forming a bicyclic aromatic ring together with B.

In one aspect of the present invention, the bicyclic aromatic ring formed by A and B may be selected from the following structures:

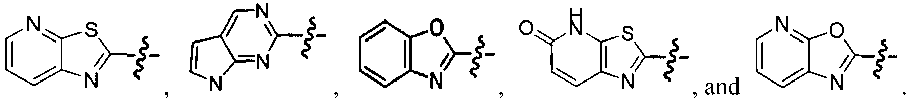

In one aspect of the present invention, R₅ may be phenyl substituted with methoxy, -(C=O)NH₂, or -OCF₃, or pyridinonyl.

In one aspect of the present invention, D may be phenyl, carboxyl, benzoimidazolyl, benzoxazolyl, or isoindolinyl.

In one aspect of the present invention, when D is aryl and A is not pyrrole or pyridone, E may be phenyl, ethylene, -C≡C-, pyrrolidinyl, pyrrolidinylcarbonyl, methylaminocarbonyl, furanyl, cyclobutylamino, pyrrolyl, cyclobutyl, or azetidinyl, wherein R₆, R₇ are each independently absent, hydrogen, carboxyl, diethylaminocarbonyl, methylaminocarbonyl, aminocarbonyl, -Si(CH₃)₃, or isopropyl.

In one aspect of the present invention, when D is aryl and A is pyrrole or pyridone, E may be halo or -C≡C-, wherein R₆, R₇ are each independently absent, hydrogen, Si(CH₃)₃, or carboxyl.

In one aspect of the present invention, when D is benzoimidazolyl, benzoxazolyl, or isoindolinyl, E may be hydrogen, -B(OH)₂, carboxyl, aminocarbonyl, aminosulfonyl, or methylene, wherein R₆, R₇ are each independently absent or carboxyl.

In one aspect of the present invention, the compound of Chemical Formula 1 may be selected from the group consisting of:
N-([1,1'-biphenyl]-4-ylmethyl)-N-(4-methoxyphenethyl)thiazolo[5,4-b]pyridin-2-amine,
3-(4-(((7-fluorobenzo[d]thiazol-2-yl)(4-methoxyphenethyl)amino)-methyl)phenyl)propanoic acid,
N,N-diethyl-3-(4-(((7-fluorobenzo[d]thiazol-2-yl)(4-methoxyphenethyl)amino)methyl)phenyl)propiolamide,
3-(4-(((7-fluorobenzo[d]thiazol-2-yl)(4-methoxyphenethyl)amino)-methyl)phenyl)-N-methylpropiolamide,
3-(4-(((7-fluorobenzo[d]thiazol-2-yl)(4-methoxyphenethyl)amino)-methyl)phenyl)propiolamide,
N-(4-methoxyphenethyl)-7-methyl-7H-pyrrolo[2,3-d]pyrimidin-2-amine,
N-(4-bromobenzyl)-N-(4-methoxyphenethyl)-7-methyl-7H-pyrrolo[2,3-d]pyrimidin-2-amine,
7-fluoro-N-(4-methoxyphenethyl)-N-(3-((trimethylsilyl)ethynyl)-benzyl)benzo[d]thiazol-2-amine,
N-(3-ethynylbenzyl)-7-fluoro-N-(4-methoxyphenethyl)benzo[d]thiazol-2-amine,
N-(4-methoxyphenethyl)-7-methyl-N-(4-((trimethylsilyl)ethynyl)-benzyl)-7H-pyrrolo[2,3-d]pyrimidin-2-amine,
N-(4-ethynylbenzyl)-N-(4-methoxyphenethyl)-7-methyl-7H-pyrrolo[2,3-d]pyrimidin-2-amine,
3-(4-(((4-methoxyphenethyl)(7-methyl-7H-pyrrolo[2,3-d]pyrimidin-2-yl)amino)methyl)phenyl)propiolic acid,
1-(4-(((7-fluorobenzo[d]thiazol-2-yl)(4-methoxyphenethyl)amino)-methyl)phenyl)pyrrolidine-3-carboxylic acid,
(4-(((7-fluorobenzo[d]thiazol-2-yl)(4-methoxyphenethyl)amino)-methyl)benzoyl)-L-proline,
1-(4-(((7-fluorobenzo[d]thiazol-2-yl)(4-methoxyphenethyl)amino)-methyl)benzoyl)pyrrolidine-3-carboxylic acid,
(4-(((7-fluorobenzo[d]thiazol-2-yl)(4-methoxyphenethyl)amino)-methyl)benzoyl)glycine,
1-(3-(((7-fluorobenzo[d]thiazol-2-yl)(4-methoxyphenethyl)amino)-methyl)phenyl)pyrrolidine-3-carboxylic acid,
(4-(((7-fluorobenzo[d]thiazol-2-yl)(4-methoxyphenethyl)amino)-methyl)benzoyl)-D-valine,
(4-(((7-fluorobenzo[d]thiazol-2-yl)(4-methoxyphenethyl)amino)-methyl)phenyl)-L-proline,
(3-(((7-fluorobenzo[d]thiazol-2-yl)(4-methoxyphenethyl)amino)-methyl)phenyl)-L-proline,
(2-(((7-fluorobenzo[d]thiazol-2-yl)(4-methoxyphenethyl)amino)-methyl)-1-methyl-1H-benzo[d]imidazol-6-yl)boronic acid,
(S)-1-(3-(((7-fluorobenzo[d]thiazol-2-yl)(4-methoxyphenethyl)amino)-methyl)phenyl)pyrrolidine-3-carboxylic acid,
(R)-1-(3-(((7-fluorobenzo[d]thiazol-2-yl)(4-methoxyphenethyl)amino)-methyl)phenyl)pyrrolidine-3-carboxylic acid,
(R)-1-(4-(((7-fluorobenzo[d]thiazol-2-yl)(4-methoxyphenethyl)-amino)methyl)phenyl)pyrrolidine-3-carboxylic acid,
(S)-1-(4-(((7-fluorobenzo[d]thiazol-2-yl)(4-methoxyphenethyl)amino)-methyl)phenyl)pyrrolidine-3-carboxylic acid,
5-(4-(((7-fluorobenzo[d]thiazol-2-yl)(4-methoxyphenethyl)amino)-methyl)phenyl)furan-2-carboxylic acid,
2-(((7-fluorobenzo[d]thiazol-2-yl)(4-methoxyphenethyl)amino)methyl)-1-methyl-1H-benzo[d]imidazole-6-carboxylic acid,
2-(((7-fluorobenzo[d]thiazol-2-yl)(4-methoxyphenethyl)amino)-methyl)benzo[d]oxazole-5-carboxylic acid,
7-fluoro-N-(isoindolin-5-ylmethyl)-N-(4-methoxyphenethyl)benzo-[d]thiazol-2-amine,
5-(((7-fluorobenzo[d]thiazol-2-yl)(4-methoxyphenethyl)amino)-methyl)isoindoline-2-carboxamide,
5-(((7-fluorobenzo[d]thiazol-2-yl)(4-methoxyphenethyl)amino)-methyl)isoindoline-2-sulfonamide,
2-(5-(((7-fluorobenzo[d]thiazol-2-yl)(4-methoxyphenethyl)amino)-methyl)isoindolin-2-yl)acetic acid,
1-((4-(((7-fluorobenzo[d]thiazol-2-yl)(4-methoxyphenethyl)amino)-methyl)phenyl)amino)cyclobutane-1-carboxylic acid,
1-(4-(((7-fluorobenzo[d]thiazol-2-yl)(4-methoxyphenethyl)amino)-methyl)phenyl)-1H-pyrrole-3-carboxylic acid,
N-(7-fluorobenzo[d]thiazol-2-yl)-N-(4-methoxyphenethyl)glycine,
1-(4-(((7-fluorobenzo[d]thiazol-2-yl)(4-methoxyphenethyl)amino)-methyl)phenyl)cyclobutane-1-carboxylic acid,
(R)-1-(4-((benzo[d]oxazol-2-yl(4-methoxyphenethyl)amino)methyl)-phenyl)pyrrolidine-3-carboxylic acid,
(S)-1-(4-((benzo[d]oxazol-2-yl(4-methoxyphenethyl)amino)methyl)-phenyl)pyrrolidine-3-carboxylic acid,
1-(4-(((7-fluorobenzo[d]thiazol-2-yl)(4-methoxyphenethyl)amino)-methyl)phenyl)azetidine-3-carboxylic acid,
(R)-1-(3-(((7-fluorobenzo[d]thiazol-2-yl)(4-(trifluoromethoxy)-phenethyl)amino)methyl)phenyl)pyrrolidine-3-carboxylic acid,
(3R)-1-(3-(1-((7-fluorobenzo[d]thiazol-2-yl)(4-methoxyphenethyl)-amino)ethyl)phenyl)pyrrolidine-3-carboxylic acid,
(3S)-1-(4-(1-((7-fluorobenzo[d]thiazol-2-yl)(4-methoxyphenethyl)-amino)ethyl)phenyl)pyrrolidine-3-carboxylic acid,
(S)-1-(4-(((4-methoxyphenethyl)(4,5,7-trifluorobenzo[d]thiazol-2-yl)amino)methyl)phenyl)pyrrolidine-3-carboxylic acid,
3-(4-(((4-methoxyphenethyl)(oxazolo[5,4-b]pyridin-2-yl)amino)-methyl)phenyl)propiolic acid,
3-(4-(((4-methoxyphenethyl)(5-oxo-4,5-dihydrothiazolo[5,4-b]pyridin-2-yl)amino)methyl)phenyl)propiolic acid,
1-(4-(((7-fluorobenzo[d]thiazol-2-yl)(4-methoxyphenethyl)amino)-methyl)phenyl)piperidine-4-carboxylic acid,
1-(4-(((7-fluorobenzo[d]thiazol-2-yl)(4-methoxyphenethyl)amino)-methyl)phenyl)-1H-pyrazole-4-carboxylic acid,
(S)-1-(4-(((4-carbamoylphenethyl)(7-fluorobenzo[d]thiazol-2-yl)amino)methyl)phenyl)pyrrolidine-3-carboxylic acid, and
(S)-1-(4-(((7-fluorobenzo[d]thiazol-2-yl)(2-(2-oxopyridin-1(2H)-yl)ethyl)amino)methyl)phenyl)pyrrolidine-3-carboxylic acid.

The present invention also provides a pharmaceutical composition for preventing or treating immune cell migration-related diseases comprising the compound, an isomer thereof, or a pharmaceutically acceptable salt thereof as an active ingredient.

In the present invention, the term "immune cell" refers to cells involved in immune responses in the body, and includes those known as immune cells in the art, particularly those known as immune cells present in the human body, without being particularly limited in type. Examples include monocytes, macrophages, neutrophils, eosinophils, basophils, dendritic cells, natural killer cells, megakaryocytes, T cells, and B cells. Preferably, it may refer to monocytes, macrophages, or neutrophils. Immune cells express KARS1.

In the present invention, the term "immune cell migration-related disease" refers to diseases in which excessive immune cell migration (and/or infiltration) is known in the art to be a major pathogenic mechanism, without being particularly limited in specific types. Examples may include cardiovascular diseases, fibrotic diseases, inflammatory diseases, and Alport syndrome.

The cardiovascular diseases are not particularly limited in specific types, but examples may include hypertension (including inflammatory complications caused by hypertension), pulmonary arterial hypertension, atherosclerosis, angina pectoris, myocardial infarction, ischemic cerebrovascular disease, arteriolosclerosis, and medial sclerosis.

The fibrotic diseases are not particularly limited in specific types, but examples may include scleroderma, rheumatoid arthritis, Crohn's disease, ulcerative colitis, myelofibrosis, pulmonary fibrosis, hepatic fibrosis, liver cirrhosis, kidney fibrosis, glomerulosclerosis, myofibrosis, cardiac fibrosis, interstitial fibrosis, pancreatic fibrosis, splenic fibrosis, mediastinal fibrosis, vascular fibrosis, skin fibrosis, ocular fibrosis, macular degeneration, joint fibrosis, thyroid fibrosis, endomyocardial fibrosis, peritoneal fibrosis, retroperitoneal fibrosis, progressive massive fibrosis, nephrogenic systemic fibrosis, systemic lupus erythematosus, hereditary fibrosis, infectious fibrosis, irritant fibrosis, fibrosis due to chronic autoimmunity, fibrosis due to antigen incompatibility in organ transplantation, fibrous complications of surgery, fibrosis due to hyperlipidemia, fibrosis due to obesity, diabetic fibrosis, fibrosis due to hypertension, and occlusion due to fibrosis during stent insertion.

In the present invention, the inflammatory disease is not particularly limited in its type, but preferably includes autoimmune diseases, inflammatory bowel diseases, dermatitis (e.g., atopic dermatitis, eczema, psoriasis, etc.), diabetic eye diseases (e.g., diabetic retinopathy), peritonitis, osteomyelitis, cellulitis, meningitis, encephalitis, pancreatitis, trauma-induced shock, bronchial asthma, rhinitis, sinusitis, otitis media, pneumonia, gastritis, enteritis, cystic fibrosis, stroke (e.g., cerebral stroke), bronchitis, bronchiolitis, hepatitis (e.g., cirrhosis, non-alcoholic steatohepatitis), nephritis (e.g., diabetic renal failure), proteinuria, arthritis (e.g., psoriatic arthritis, osteoarthritis), neuritis (e.g., diabetic neuropathy, multiple sclerosis), gout, spondylitis, Reiter's syndrome, polyarteritis nodosa, vasculitis, amyotrophic lateral sclerosis, Wegener's granulomatosis, hypercytokinemia, polymyalgia rheumatica, giant cell arteritis, calcium crystal deposition arthropathy, pseudogout, non-articular rheumatism, bursitis, tendinitis, epicondylitis (e.g., tennis elbow), neuropathic joint disease (e.g., Charcot's joint), hemarthrosis, Henoch-Schönlein purpura, hypertrophic osteoarthropathy, multicentric reticulohistiocytosis, surcoilosis, hemochromatosis, sickle cell disease, hyperlipoproteinemia, hypogammaglobulinemia, hyperparathyroidism, acromegaly, familial Mediterranean fever, Behcet's disease, systemic lupus erythematosus, relapsing fever, psoriasis, multiple sclerosis, sepsis, septic shock, acute respiratory distress syndrome, multiple organ failure, chronic obstructive pulmonary disease, acute lung injury, and broncho-pulmonary dysplasia, and may also include chronic inflammatory diseases.

In the present invention, the autoimmune disease may be selected from the group consisting of rheumatoid arthritis, systemic scleroderma, systemic lupus erythematosus, psoriasis, asthma, ulcerative colitis, Behcet's disease, Crohn's disease, multiple sclerosis, dermatomyositis, collagen disease, vasculitis, arthritis, granulomatosis, organ-specific autoimmune lesions, ulcerative colitis, and graft-versus-host disease (GvHD).

The chronic inflammatory disease in the present invention refers to a state in which the aforementioned types of inflammatory diseases have become chronic, and preferred examples thereof include asthma, atopic dermatitis, eczema, psoriasis, osteoarthritis, gout, psoriatic arthritis, cirrhosis, non-alcoholic steatohepatitis, chronic obstructive pulmonary disease, rhinitis, diabetic retinopathy, diabetic renal failure, diabetic neuropathy, and multiple sclerosis, but are not limited thereto.

The pharmaceutical composition according to the present invention may include the compound of the present invention alone or may be formulated in an appropriate form together with one or more pharmaceutically acceptable carriers and may further contain excipients or diluents. In the above, "pharmaceutically acceptable" refers to a non-toxic composition that is physiologically acceptable and does not cause allergic reactions such as gastrointestinal disturbances or dizziness, or similar reactions when administered to humans.

In the present invention, the content of the composition is not significantly limited depending on the purpose or aspect of use, and, for example, may be 0.01 to 99% by weight, preferably 0.5 to 50% by weight, and more preferably 1 to 30% by weight, based on the total weight of the composition. In addition, the pharmaceutical composition according to the present invention may further include additives such as pharmaceutically acceptable carriers, excipients, or diluents in addition to the active ingredient. The pharmaceutical composition of the present invention may include the compound represented by Formula 1 to Formula 6, prepared by the method of the present invention, in an amount of 0.1 to 99.9% by weight, and may include a carrier in an amount of 99.9% to 0.1% by weight.

Pharmaceutically acceptable carriers may include, for example, carriers for oral administration or carriers for parenteral administration. Carriers for oral administration may include lactose, starch, cellulose derivatives, magnesium stearate, stearic acid, and the like. Additionally, various drug delivery substances used for oral administration of peptide formulations may be included. Furthermore, carriers for parenteral administration may include water, suitable oils, saline, aqueous glucose, glycols, and the like, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol.

The pharmaceutical composition of the present invention may further include lubricants, wetting agents, sweeteners, flavoring agents, emulsifiers, suspending agents, and the like, in addition to the above components. Other pharmaceutically acceptable carriers and formulations may be referred to as those known in the art.

The composition of the present invention can be administered to mammals, including humans, by any method. For example, it can be administered orally or parenterally. Specifically, the route of administration of the composition of the present invention may be by injection or infusion through known administration methods, such as intravenous, intraperitoneal, intracerebral, subcutaneous, intramuscular, intraocular, intra-arterial, intrathecal, or intralesional routes, or by injection or infusion through the sustained release system described below, but is not limited thereto. For example, the compound of the present invention may be administered systemically or locally.

The pharmaceutical composition of the present invention may be formulated as an oral or parenteral preparation according to the routes of administration described above.

In the pharmaceutical composition according to the present invention, the compound may be administered in various oral and parenteral formulations during clinical administration, and when formulated, it may be prepared using diluents or excipients such as commonly used fillers, extenders, binders, wetting agents, disintegrants, surfactants, and the like. Solid preparations for oral administration include tablets, pills, powders, granules, capsules, troches, and the like, and such solid preparations may be prepared by mixing at least one excipient, for example, starch, calcium carbonate, sucrose, lactose, gelatin, and the like. In addition to simple excipients, lubricants such as magnesium stearate and talc may also be used. Liquid preparations for oral administration include suspensions, solutions, emulsions, or syrups, and may include commonly used simple diluents such as water and liquid paraffin, as well as various excipients, for example, wetting agents, sweeteners, flavoring agents, preservatives, and the like.

Preparations for parenteral administration include sterile aqueous solutions, non-aqueous solvents, suspensions, emulsions, freeze-dried preparations, and suppositories. The therapeutic composition of the present invention may be prepared in the form of a freeze-dried cake or an aqueous solution by mixing a compound with a desired purity with any physiologically acceptable carrier, excipient, or stabilizer known in the art for storage. Acceptable carriers, excipients, or stabilizers are non-toxic to the recipient at the dosages and concentrations used and include buffering agents, such as phosphates, citrates, and other organic acids; antioxidants, such as ascorbic acid; low molecular weight (about 10 residues or fewer) polypeptides; proteins, such as serum albumin, gelatin, or immunoglobulins; hydrophilic polymers, such as polyvinylpyrrolidone; amino acids, such as glycine, glutamine, asparagine, arginine, or lysine; monosaccharides, disaccharides, and other carbohydrates, such as glucose, mannose, or dextrins; chelating agents, such as EDTA; sugar alcohols, such as mannitol or sorbitol; salt-forming counterions, such as sodium; and/or non-ionic surfactants, such as Tween, Pluronics, or polyethylene glycol (PEG).

For parenteral administration, the formulation may be prepared in the form of injections, creams, lotions, topical ointments, oils, moisturizers, gels, aerosols, and nasal inhalants by methods known in the art. These formulations are known in the art.

The total effective amount of the compound of the present invention may be administered as a single dose or may be administered by a fractionated treatment protocol in which multiple doses are administered over a prolonged period. The pharmaceutical composition of the present invention may vary the content of the active ingredient (the compound of the present invention) depending on the severity of the disease and/or the purpose, but typically, it can be administered several times a day in an effective dose of 0.01 µg to 10000 mg, preferably 0.1 µg to 1000 mg, per dose. However, the dosage of the pharmaceutical composition is determined by considering various factors such as the formulation method, route of administration, frequency of treatment, as well as the patient's age, weight, health condition, gender, severity of the disease, diet, and excretion rate, so the effective dose for the patient is determined accordingly. Considering these factors, one skilled in the art will be able to determine the appropriate effective dose of the composition of the present invention. The pharmaceutical composition according to the present invention is not particularly limited in its formulation, route of administration, or method of administration, as long as it exhibits the effects of the present invention.

The present invention also provides a pharmaceutical composition for preventing or inhibiting cancer metastasis, comprising the compound, its isomer, or a pharmaceutically acceptable salt thereof as an active ingredient.

The cancer is not particularly limited as long as it is known as a malignant tumor in the art, and it may be selected from the group consisting of breast cancer, colon cancer, lung cancer, small cell lung cancer, gastric cancer, liver cancer, blood cancer, bone cancer, pancreatic cancer, skin cancer, head or neck cancer, melanoma of the skin or intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvis carcinoma, CNS tumor, primary CNS lymphoma, spinal tumor, brainstem glioma, and pituitary adenoma.

The present invention also provides the use of the compound, its isomer, or a pharmaceutically acceptable salt thereof for manufacturing a composition for treating immune cell migration-related diseases.

The present invention also provides a method for treating immune cell migration-related diseases, comprising administering an effective amount of a composition comprising the compound, its isomer, or a pharmaceutically acceptable salt thereof as an active ingredient to a subject in need thereof.

The present invention also provides the use of the compound, its isomer, or a pharmaceutically acceptable salt thereof for manufacturing a composition for inhibiting cancer metastasis.

The present invention also provides a method for inhibiting cancer, comprising administering an effective amount of a composition comprising the compound, its isomer, or a pharmaceutically acceptable salt thereof as an active ingredient to a subject in need thereof.

The term "effective amount" of the present invention refers to an amount that, when administered to a subject, exhibits an effect of improving, treating, detecting, diagnosing, or inhibiting or reducing cancer. The term "subject" may include animals, preferably mammals, particularly humans, and may also include cells, tissues, or organs derived from animals. The subject may be a patient in need of the effect.

The term "treatment" of the present invention comprehensively refers to improving symptoms caused by cancer or the disease, and it may include curing the disease, substantially preventing it, or improving the condition. It may also include alleviating, curing, or preventing one or more symptoms or most symptoms caused by the disease, but is not limited thereto.

The term "comprising" as used herein is used in the same sense as "including" or "characterized by," and in the composition or method according to the present invention, it does not exclude additional components or steps of methods that are not specifically mentioned. The term "consisting of" means excluding additional elements, steps, or components that are not separately described. The term "consisting essentially of" means that, within the scope of the composition or method, it may include substances or steps that do not substantially affect the basic characteristics of the described substances or steps.

### [Effect of the Invention]

The novel compound of the present invention can regulate the migration of immune cells and cancer cells by inhibiting the activity of KARS1, thereby exhibiting very remarkable effects in the prevention, improvement, and treatment of diseases related to immune cell migration and cancer metastasis.

### [Brief Description of the Drawings]

Figure 1 is the result of evaluating the inhibitory ability of Compound 25 (C0047) and Compound 43 (C0216) on the migration of KARSI-induced Raw264.7 cells.
Figure 2 is the result of evaluating the inhibitory ability on cancer cell migration after treating cancer cells with Compound 25 (C0047) and Compound 43 (C0216) according to the present invention (LN: laminin 421).
Figure 3 shows the 10 mpk rat po PK (pharmacokinetic) results of Compound 25 (C0047), Compound 41 (C0189), Compound 42 (C0190), and Compound 43 (C0216) according to the present invention.
Figure 4 shows the 30 mpk rat po PK results of Compound 25 (C0047), Compound 41 (C0189), and Compound 43 (C0216) according to the present invention.

### [Detailed Description of the Invention]

Hereinafter, the present invention will be described in detail with reference to the following examples. However, the following examples are merely illustrative of the present invention, and the present invention is not limited thereto.

### Example 1: Preparation of Compounds

In the present invention, the compound of Formula 1 was prepared according to the following Synthesis Methods 1 to 4:

### Synthesis Method 1.

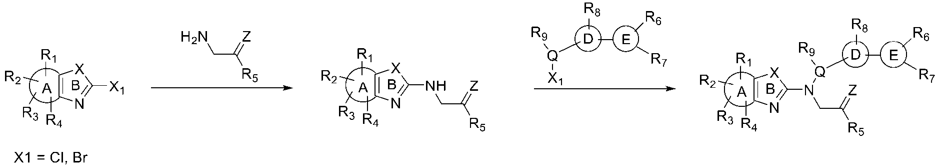

### Synthesis Method 2.

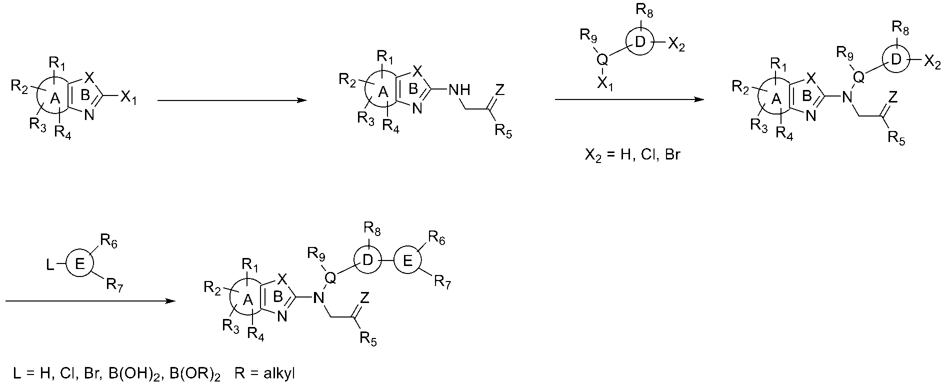

### Synthesis Method 3.

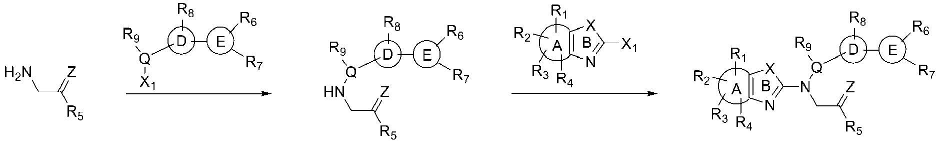

### Synthesis Method 4.

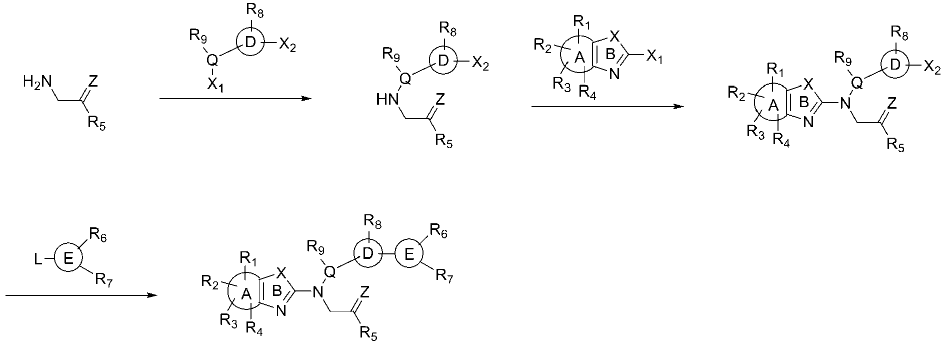

In the above synthesis methods, A, B, Q, D, E, Z, and R₁ to R₁₀ are as defined above.

Specific examples, structures, and NMR analysis results of the compound of Formula 1 prepared according to the above methods are specifically shown in Tables 1 and 2 below.

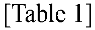

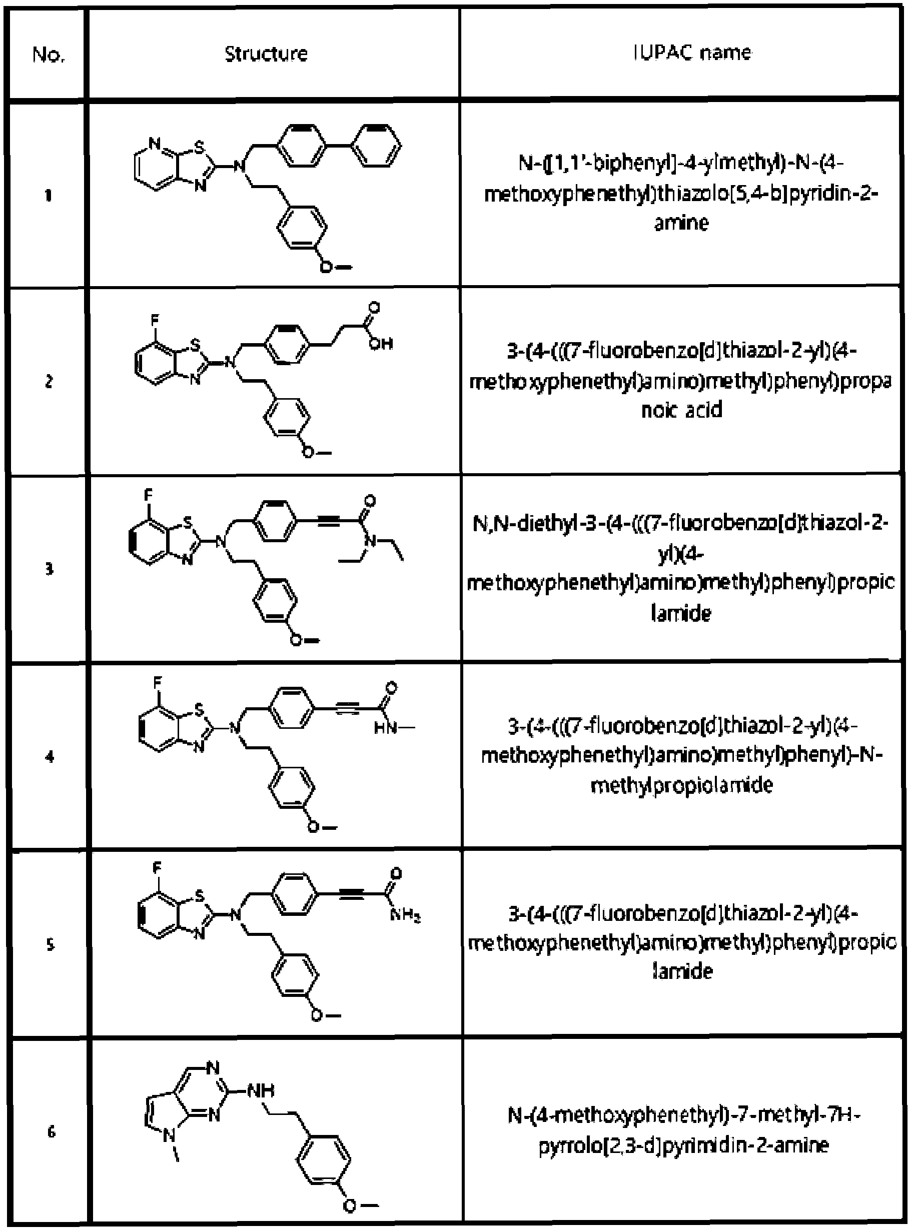

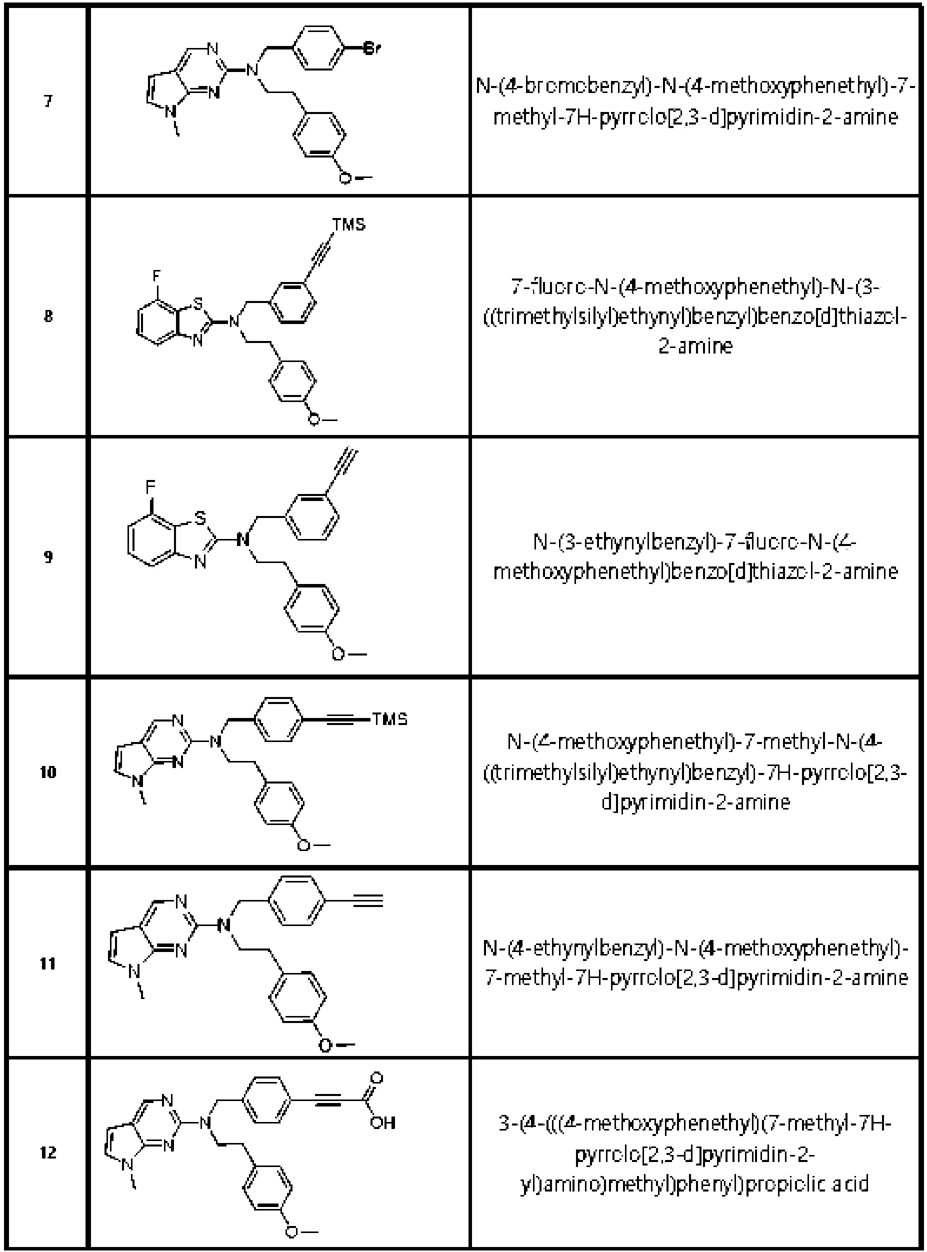

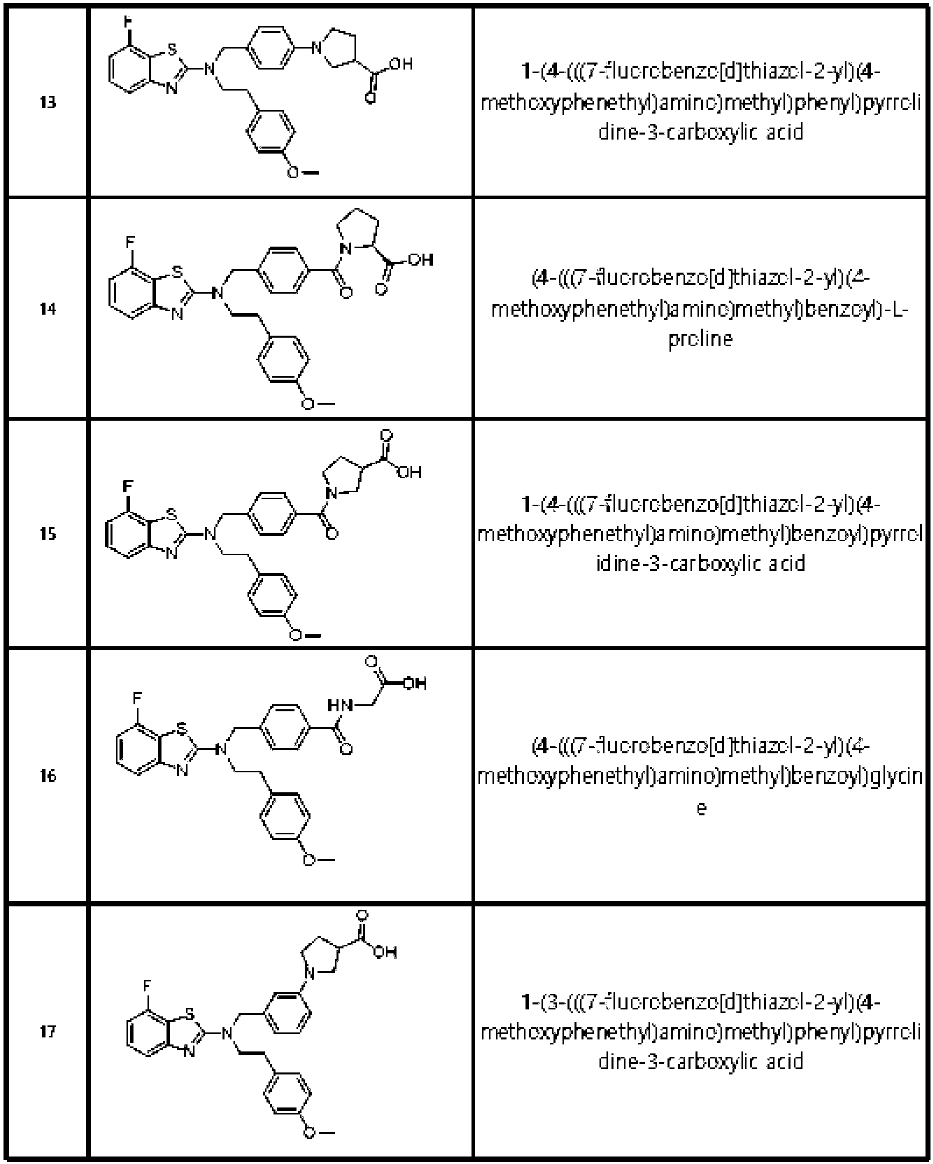

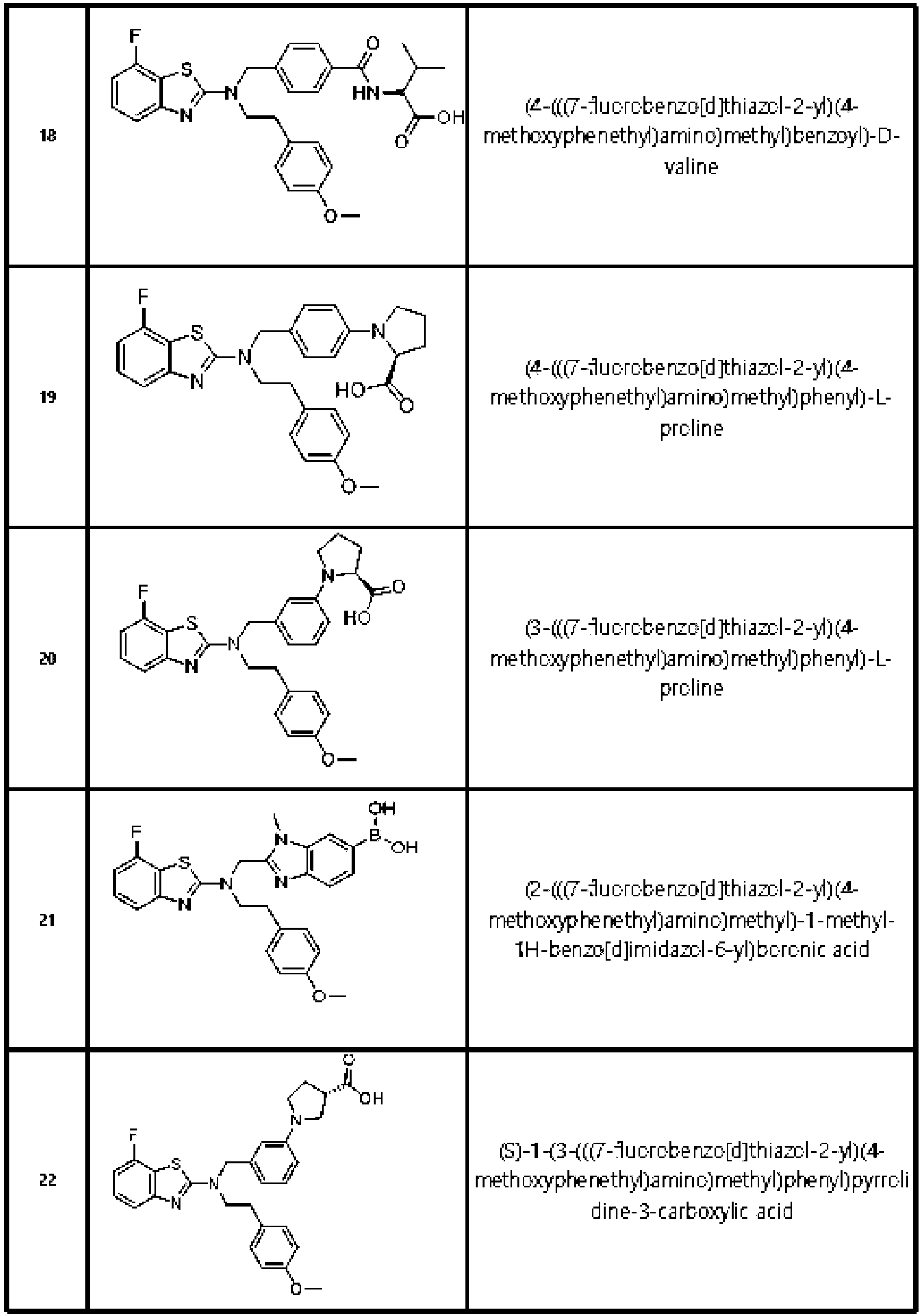

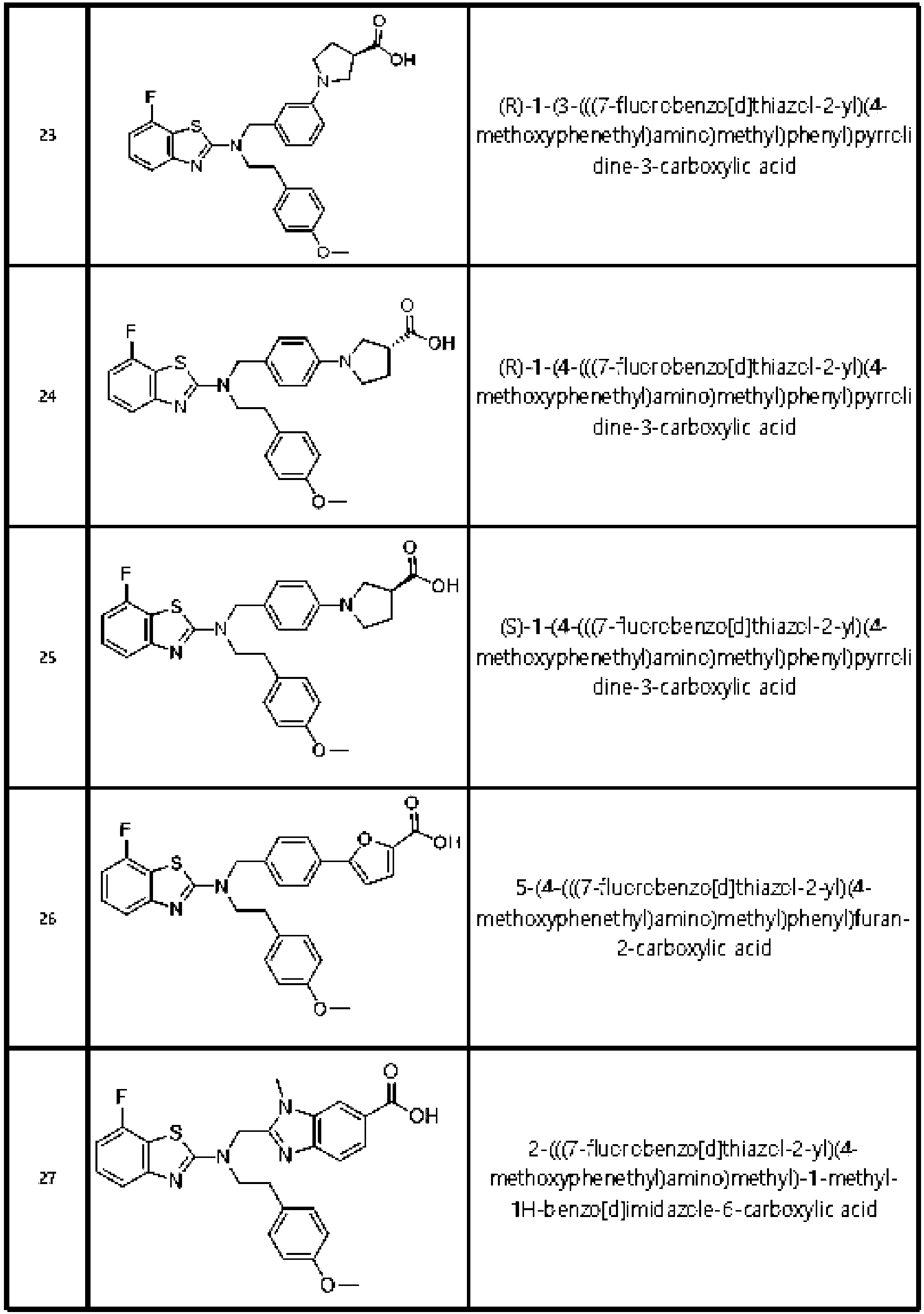

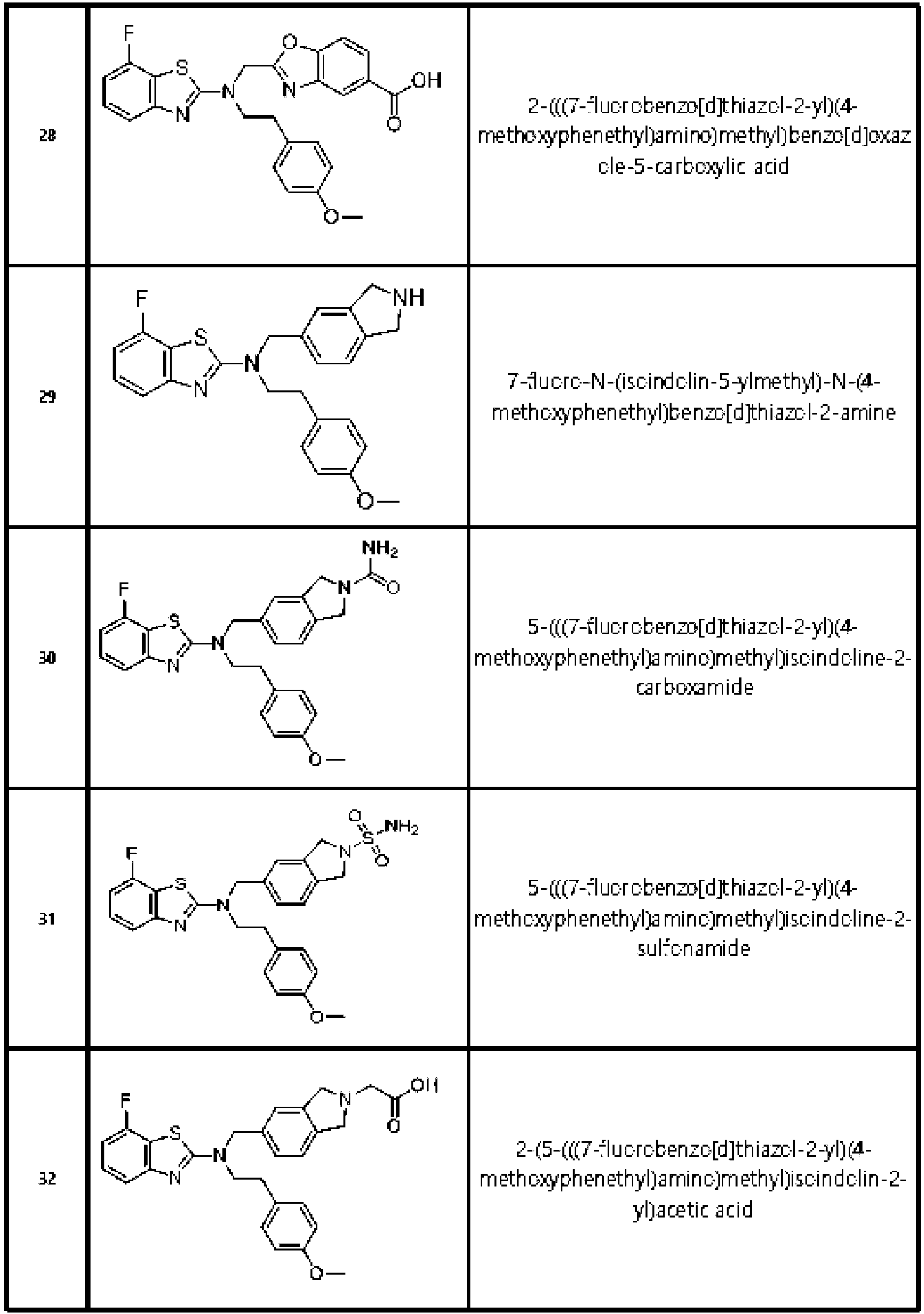

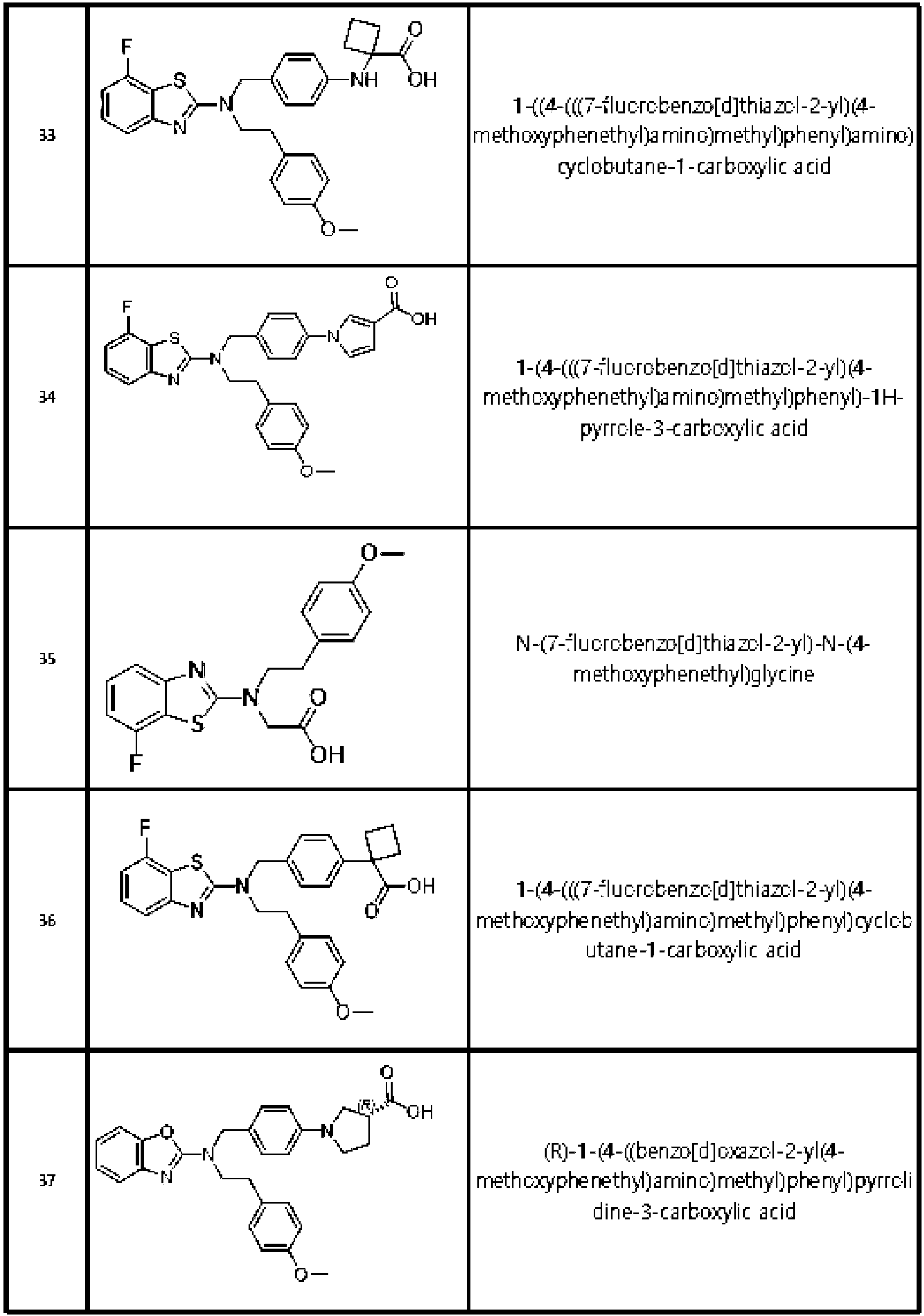

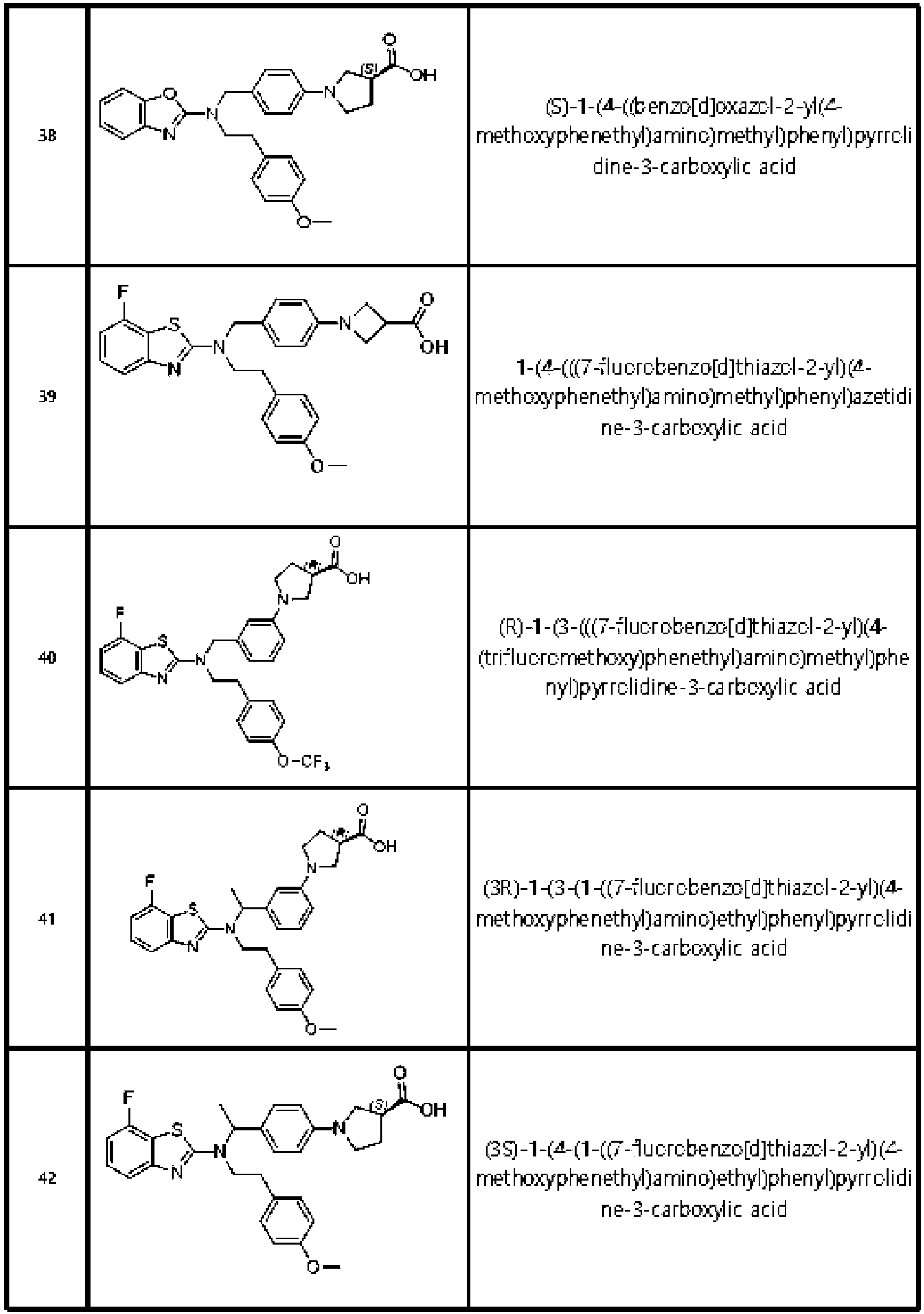

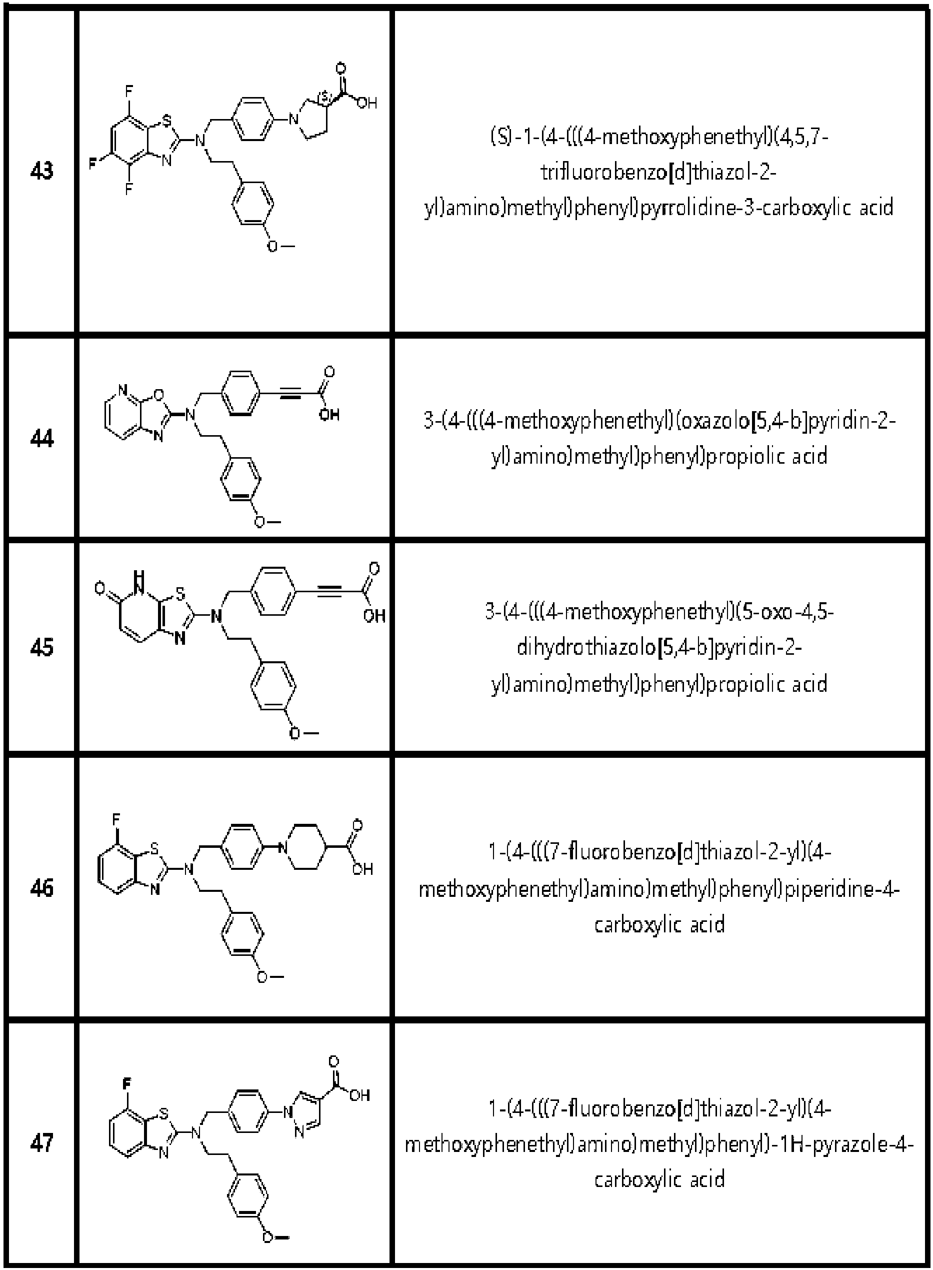

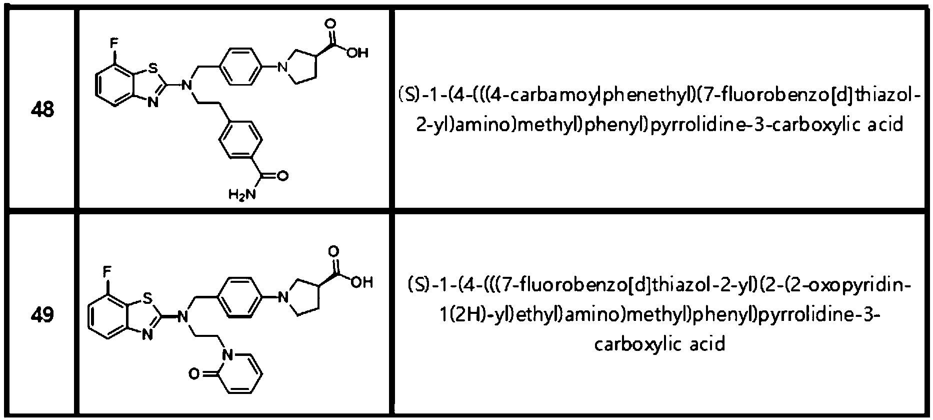

**[Table 2]**

| **No.** | **1H NMR** |
|---|---|
| 1 | - |
| 2 | ¹H NMR (600 MHz, CHLOROFORM-*D*) δ 7.34 (dd, *J* = 8.1, 0.9 Hz, 1H), 7.25-721 (m, 2H), 7.16-7.13 (m, 4H), 7.09-7.06 (m, 2H), 6.83-6.80 (m, 2H), 6.78-6.77 (m, 1H), 458 (s, 2H), 3.77 (s, 3H), 3.62 (t, J = 7.7 Hz, 2H), 2.90 (td, *J* = 7.8, 4.6 Hz, 4H), 2.63 (t, J = 7.8 Hz, 2H), 3.79 (s, 3H), 3.64 (t, *J* = 7.5 Hz, 2H), 2.93 (dd, *J* = 15.3, 6.3 Hz, 5H). |
| 3 | 1H NMR (600 MHz, CHLOROFORM-D) δ 7.49 (d, J = 8.4 Hz, 2H), 7.37 (dd, J = 8.1, 0.9 Hz, 1H), 7.28 - 726 (m, 1H), 7.26 - 7.23 (m, 2H), 7.12 - 7.08 (m, 2H), 6.87 - 6.79 (m, 3H), 4.66 (s, 2H), 3.79 (s, 3H), 3.70 - 3.61 (m, 4H), 3.47 (q, J = 7.1 Hz, 2H), 2.96 - 2.92 (m, 2H), 1.31 - 1.23 (m, 6H). |
| 4 | ¹H NMR (600 MHz, CHLOROFORM-*D*) δ 7.77 (dd *J* = 8.2, 0.8 Hz, 1H), 7.47 - 7.45 (m, 2H), 7.37 - 7.35 (m, 1H), 7.28 - 7.26 (m, 1H), 725 (d *J* = 2.3 Hz, 1H), 7.24 - 722 (m, 2H), 7.17 - 7.14 (m, 1H), 7.11 - 7.08 (m, 2H), 6.85 - 6.83 (m, 2H), 5.94 (d, *J* = 5.9 Hz, 1H), 4.65 (s, 2H), 3.79 (s, 3H), 3.64 (t, *J* = 7.5 Hz, 2H), 2.93 (dd, *J* = 153, 6.3 Hz, 5H). |
| 5 | ¹H NMR (600 MHz, CHLOROFORM-*D*) δ 8.05 (dt, *J* = 8.4, 0.9 Hz, 1H), 7.79 - 7.70 (m, 2H), 7.58 - 7.54 (m, 1H), 7.47 - 7.42 (m, 1H), 736 (dd, *J* = 8.1, 0.9 Hz, 2H), 7.14 - 7.08 (m, 2H), 6.88 - 6.79 (m, 3H), 5.31 (s, 1H), 4.70 (d, *J* = 4.4 Hz, 2H), 3.79 (d, *J* = 3.5 Hz, 3H), 3.67 (dt, *J* = 12.6, 7.4 Hz, 2H), 2.94 (d, *J* = 8.4 Hz, 2H). |
| 6 | ¹H NMR (600 MHz, CHLOROFORM-*D*) δ 8.48 (t, *J* = 1.0 Hz, 1H), 7.20 - 7.16 (m, 2H), 6.88 - 6.84 (m, 2H), 6.80 (d, *J* = 3.6 Hz, 1H), 6.32 (d, *J* = 3.6 Hz, 1H), 5.19 (s, 1H), 3.79 (d, *J* = 0.6 Hz, 3H), 3.73 - 3.69 (m, 5H), 2.90 (t, *J* = 7.1 Hz, 2H). |
| 7 | ¹H NMR (600 MHz, CHLOROFORM-*D*) δ 8.58 (s. 1H), 7.40 - 7.36 (m, 2H), 7.16 - 7.12 (m, 4H), 6.84 - 6.79 (m, 3H), 6.33 (d, *J* = 3.5 Hz, 1H), 4.81 (s, 2H), 3.79 (s, 5H), 3.69 (s, 3H), 2.93 - 2.87 (m, 2H). |
| 8 | ¹H NMR (600 MHz. CHLOROFORM-*D*) δ 7.40 - 7.37 (m, 2H), 7.33 (td, *J* = 1.7, 0.7 Hz. 1H), 7.29 - 7.26 (m, 1H), 7.25 (dd, *J* = 1.4, 0.9 Hz, 1H), 7.20 (ddd, *J* = 7.7, 1.9, 1.2 Hz, 1H), 7.11 (d. *J* = 8.6 Hz, 2H), 6.85 (d, *J* = 8.6 Hz, 2H), 6.84 - 6.81 (m, 1H), 4.61 (s, 2H), 3.80 (s, 3H), 3.65 (t, *J* = 7.6 Hz, 2H), 2.95 - 2.90 (m, 2H), 0.24 (s, 9H). |
| 9 | 1H NMR (600 MHz, CHLOROFORM-D) δ 7.41 (dt. J = 7.5, 1.5 Hz, 1H), 7.38 - 7.36 (m, 2H), 7.30 - 7.26 (m, 2H), 7.25 (dd, J = 6.0, 1.5 Hz, 1H), 7.11 (d, J = 8.6 Hz, 2H), 6.85 (d, J = 8.6 Hz. 2H), 6.83 (dt, J = 9.2. 1.1 Hz. 1H), 4.62 (s, 2H), 3.80 (s, 3H), 3.65 (t, J = 7.5 Hz, 2H), 2.96 - 2.92 (m, 2H). |
| 10 | ¹H NMR (600 MHz, CHLOROFORM-*D*) δ 7.41 (dt, *J* = 7.5, 1.5 Hz, 1H), 7.38 - 7.36 (m, 2H), 7.30 - 7.26 (m, 2H), 7.25 (dd, *J* = 6.0, 1.5 Hz, 1H), 7.11 (d. *J* = 8.6 Hz, 2H), 6.85 (d, *J* = 8.6 Hz, 2H), 6.83 (dt, *J* = 9.2, 1.1 Hz, 1H), 4.62 (s, 2H), 3.80 (s, 3H), 3.65 (t, *J* = 7.5 Hz, 2H), 2.96 - 2.92 (m, 2H). |
| 11 | ¹H NMR (600 MHz, CHLOROFORM-*D*) δ 8.58 (d, *J* = 3.6 Hz, 1H), 7.56 - 7.50 (m, 1H), 7.43 - 7.36 (m, 2H), 7.30 (s, 1H), 7.23 - 7.18 (m, 2H), 7.14 (dd, *J* = 8.8, 2.4 Hz 2H), 6.85 - 6.78 (m, 3H), 6.34 (d. *J* = 3.5 Hz, 1H), 4.86 (d, *J* = 4.1 Hz, 2H), 3.79 (s, 5H), 3.68 (d. *J* = 2.2 Hz, 3H), 2.93 - 2.88 (m, 2H). |
| 13 | 1H NMR (600 MHz, CHLOROFORM-*D*) δ 8.04-8.02 (m, 1H), 7.39-7.32 (m, 2H), 7.26-7.22 (m, 2H), 7.21-7.09 (m, 5H), 6.96-6.77 (m, 5H), 6.53-6.50 (m, 2H), 4.53 (s, 2H), 3.88 (s, 5H), 3.66-3.52 (m, 3H), 3.44-3.40 (m, 2H), 3.34-3.23 (m, 3H), 2.97-2.89 (m, 3H), 2.34-2.28 (m, 2H) |
| 15 | ¹H NMR (600 MHz. Methanol-*d4*) δ 7.54-7.50 (m, 2H), 7.39-7.37 (m, 2H), 7.33-7.25 (m, 2H), 7.16-7.13 (m, 2H), 6.86-6.82 (m, 3H), 4.75 (s, 2H), 3.79-3.41 (m, 9H), 3.00-2.85 (m, 3H), 2.20-2.06 (m, 2H) |
| 16 | ¹H NMR (600 MHz, DMSO-d6) δ 8.50 (s, 1H), 7.84 (d. J = 12Hz, 2H), 7.41 (d, J = 12Hz, 2H), 7.32-7.29 (m, 2H), 7.18 (d, J = 12Hz, 2H), 6.95 (t, *J* = 12 Hz, 1H), 6.86 (d, J = 12Hz, 2H), 4.83 (s, 2H), 3.78 (d, *J* = 8.2 Hz, 2H), 3.71 (s, 5H), 3.16 (s, 1H), 2.92 - 2.88 (m, 2H). |
| 17 | 1H NMR (600 MHz, CHLOROFORM-*D*) δ 7.39-7.36 (m, 1H), 7.27-7.10 (m, 4H), 6.85-6.78 (m, 3H), 6.58 (d, J = 8.0 Hz, 1H), 6.49 (d, J = 8.0 Hz, 1H), 6.42 (s, 1H), 4.58 (s, 2H), 3.78 (s, 3H), 3.71-3.69 (m, 2H), 3.53 (d, J = 8.0 Hz, 2H), 3.40-3.18 (m, 3H), 2.94 (t, J = 8.0 Hz, 2H), 2.31-2.26 (m, 2H) |
| 21 | ¹H NMR (400 MHz, DMSO) δ 8.25 (s, 1H), 7.92 (d, *J =* 9.3 Hz, 1H), 7.67 (d, *J* = 8.2 Hz, 1H), 7.37 - 7.28 (m, 2H), 7.28 - 7.19 (m, 2H), 7.03 (ddd, *J* = 9.3, 6.3, 2.9 Hz, 1H), 6.BB - 6.80 (m, 2H), 5.42 (s, 2H), 4.04 (s, 3H), 3.B5 , *J* = 7.7 Hz, 2H), 3.68 (s, 3H), 3.00 (t. *J* = 7.5 Hz 2H). |
| 22 | H NMR (400 MHz, CDCl₃) δ 7.39 (d, *J* = 8.0 Hz, 1H), 7.23 (d, *J* = 8.0 Hz, 1H), 7.18 (t. *J* = 7.9 Hz, 1H), 7.11 (d. *J* = 8.5 Hz, 2H), 6.84 (d, *J* = 8.6 Hz, 2H), 6.79 (d, *J* = B.6 Hz, 1H), 6.59 (d, *J* = 7.5 Hz, 1H), 6.50 (d, *J* = B.7 Hz, 1H), 6.43 (s, 1H), 4.58 (s, 2H), 3.79 (s, 3H), 3.71 (q, *J* = 7.1 Hz, 2H), 3.54 (d, *J* = 7.1 Hz, 2H), 3.40 (q, *J =* 6.9 Hz, 1H), 3.31 (q, *J* = 7.7 Hz, 1H), 3.23 (t, *J* = 7.3 Hz, 1H), 2.95 (t, *J* = 7.5 Hz, 2H), 2.30 (q, *J* = 6.9 Hz, 2H). |
| 23 | ¹H NMR (400 MHz, CDCl₃) δ 7.39 (d, *J* = 8.0 Hz, 1H), 7.24 (d. *J* = 8.0 Hz, 1H), 7.18 (t. *J* = 7.8 Hz, 1H), 7.11 (d, *J* = 8.6 Hz, 2H), 6.84 (d, *J =* 8.4 Hz. 2H), 6.80 (d, *J* = 8.5 Hz. 1H), 6.59 (d, *J* = 7.5 Hz, 1H), 6.50 (d. *J* = 8.8 Hz, 1H), 6.43 (s, 1H), 4.58 (s, 2H), 3.79 (s, 3H), 3.71 (s, 2H), 3.54 (d, *J* = 7.2 Hz, 2H), 3.44 - 3.37 (m, 1H), 3.32 (q, *J* = 7.5 Hz, 1H), 3.24 (t. *J* = 7.3 Hz, 1H), 2.95 (t, *J* = 7.5 Hz, 2H), 2.30 (q, *J =* 7.0 Hz, 2H). |
| 24 | ¹H NMR (400 MHz, DMSO) δ 12.45 (s, 1H), 7.35 - 7.27 (m, 2H), 7.20 - 7.13 (m, 4H), 6.93 (ddd, *J* = 9.4, 7.4, 1.7 Hz, 1H), 6.89 - 6.84 (m, 2H), 6.53 (d, *J* = 8.6 Hz, 2H), 4.59 (s, 2H), 3.71 (s, 3H), 3.62 (t, *J* = 7.7 Hz, 2H), 3.44 - 3.36 (m, 2H), 3.28 - 3.20 (m, 2H), 3.16 (dd, *J* = 7.8, 6.4 Hz, 1H), 2.85 (t, *J* = 7.7 Hz, 2H), 2.22 - 2.09 (m, 2H). |
| 25 | ¹H NMR (400 MHz, DMSO) δ 12.40 (s, 1H), 7.31 (td, *J* = 7.6, 5.8 Hz, 2H), 7.16 (dd, *J =* 8.6, 4.3 Hz, 4H), 6.93 (ddd, *J =* 9.4, 7.4, 1.7 Hz, 1H), 6.89 - 6.83 (m, 2H), 6.52 (d, *J* = 8.5 Hz, 2H), 4.59 (s, 2H), 3.71 (s, 3H), 3.61 (d, *J* = 8.0 Hz, 2H), 3.45 - 3.37 (m, 2H), 3.28 - 3.22 (m, 2H), 3.19 - 3.14 (m, 1H), 2.85 (t, *J* = 7.6 Hz, 2H), 2.16 (dp, *J* = 19.7, 6.0 Hz, 2H). |
| 26 | ¹H NMR (400 MHz, DMSO) δ 13.13 (s, 1H), 7.82 - 7.76 (m, 2H), 7.44 (d*. J* = 8.1 Hz, 2H), 7.37 - 7.28 (m, 3H), 7.19 (d, *J* = 8.6 Hz, 2H), 7.11 (d. *J* = 3.6 Hz, 1H), 6.96 (ddd, *J =* 9.3, 7.5, 1.7 Hz, 1H), 6.87 (d, *J* = 8.6 Hz, 2H), 4.81 (s, 2H), 3.71 (s, 5H), 2.91 (t, *J* = 7.7 Hz, 2H). |
| 27 | ¹H NMR (400 MHz, DMSO) δ 8.16 (d, *J* = 1.6 Hz, 1H), 7.79 (dd, *J* = 8.4, 1.6 Hz, 1H), 7.64 (d. *J* = 8.4 Hz, 1H), 7.31 (d, *J* = 4.0 Hz, 2H), 7.18 (d, *J =* 8.2 Hz, 2H), 6.96 (ddd, *J* = 9.3. 6.1, 2.8 Hz, 1H), 6.84 (d. *J* = 8.2 Hz, 2H), 5.17 (s, 2H), 3.90 (s, 3H), 3.80 (t, *J* = 7.6 Hz, 2H), 3.69 (s, 3H), 2.95 (t, *J* = 7.5 Hz, 2H). |
| 28 | ¹H NMR (400 MHz, DMSO) δ 8.17 (s. 1H), 7.97 (dd, *J* = 8.4, 1.6 Hz, 1H), 7.11 (d, *J* = 8.4 Hz, 1H), 7.30 (t, *J* = 4.0 Hz, 2H), 7.23 (d, *J* = 8.4 Hz, 2H), 6.95-7.00 (m, 1H), 6.86 (d, *J* = 8.4 Hz, 2H), 5.16 (s, 2H), 3.86 (t, *J* = 8 Hz, 2H), 3.70 (s, 3H), 2.99 (q. *J* = 3.2 Hz, 2H) |
| 29 | ¹H NMR (400 MHz. DMSO) δ 8.21 (s, 1H), 7.33-7.27 (m, 5H), 7.18 (d, *J* = 8.4 Hz, 2H), 7.00-6.94 (m, 1H), 6.87 (d, *J* = 8.4 Hz, 2H), 4.76 (s, 2H), 4.29 (s, 4H), 3.71 (s, 3H), 3.67 (d, *J* = 7.6 Hz, 2H), 2.90 (t, *J* = 7.6 Hz, 2H) |
| 30 | ¹H NMR (400 MHz. DMSO) δ 7.34-7.30 (m, 5H), 7.17 (d. J = 8.4 Hz, 2H), 6.95-6.93 (m, 1H), 6.86 (d, *J* = 8.8 Hz, 2H), 5.92 (s, 2H), 4.76 (s, 2H), 4.53 (s, 3H), 3.71-3.64 (m, 5H), 2.97-2.85 (m, 2H) |
| 31 | ¹H NMR (400 MHz, DMSO) δ 7.35-7.26 (m, 4H), 7.18 (d, *J* = 8.8 Hz, 2H), 6.98-6.95 (m, 1H), 6.87 (d, *J* = 8.4 Hz, 1H), 4.76 (s, 2H), 4.47 (s, 2H), 3.70 (t, *J* = 4.4 Hz, 3H), 3.66 (d. *J* = 2.0 Hz, 2H), 3.42-3.39 (m, 2H), 2.92-2.88 (m, 2H) |
| 32 | H NMR (400 MHz, DMSO) δ 7.35 (d. *J* = 6.0 Hz, 1H), 7.26-7.22 (m, 1H), 7.16 (s, 2H), 711 (t, *J* = 4.4 Hz, 3H), 684 (d, *J* = 8.8 Hz, 2H), 6.80 (d. *J* = 8.8 Hz, 1H), 4.61 (s, 2H), 4.57 (d, *J* = 7.2 Hz 4H), 3.79 (s, 3H), 3.76 (s, 2H), 3.64 (t, *J* = 7.2 Hz, 2H), 2.94 (t, *J* = 7.6 Hz, 2H) |
| 33 | H NMR (400 MHz, DMSO) δ 7.33-7.26 (m, 2H), 7.16-7.13 (m, 2H), 7.05 (d, *J* = B.4 Hz, 2H), 6.95-6.91 (m, 1H), 6.86 (dd, *J =* 8.0. 2.4 Hz, 2H), 6.33 (d, *J* = B.4 Hz, 2H), 4.53 (d. *J* = 6.0 Hz, 2H), 3.71 (s, 3H), 3.61 (t, *J* = 6.B Hz, 2H), 2.85 (t, *J* = 7.6 Hz, 2H), 2.58-2.56 (m, 2H), 2.11-2.08 (m, 2H), 1.93-1.90 (m, 2H) |
| 34 | 1H NMR (400 MHz, METHANOL-d4) δ = 7.80 (t, *J* = 2.0 Hz, 1H), 7.51 (m, *J* = 8.8 Hz, 2H), 7.44 - 7.40 (m, 2H), 7.35 - 7.26 (m, 2H), 7.20 (dd, *J* = 2.4, 2.8 Hz, 1H), 7.15 (d, *J* = 8.4 Hz, 2H), 6.89 - 6.82 (m, 3H), 6.69 (dd, *J* = 2.0, 3.2 Hz, 1H), 4.75 (s, 2H), 3.77 - 3.72 (m, 5H), 2.97 (t, *J* = 7.6 Hz, 2H) |
| 36 | ¹H NMR (400 MHz, DMSO) δ 12.38-12.22 (m, 1H), 7.34-7.24 (m, 6H), 7.17 (d, *J =* 8.8 Hz, 2H), 6.97-6.93 (m, 1H), 6.86 (d, *J* = B.4 Hz, 2H), 4.73 (s, 2H), 3.71-3.66 (m, 5H), 2.90 (t, *J* = 7.6 Hz, 2H), 2.70-2.67 (m, 2H), 2.38-2.35 (m, 2H), 1.91-1.29 (m, 1H), 1.77-1.75 (m, 1H) |
| 37 | ¹H NMR (400 MHz, MeOD) δ 7.29 (t, *J* = 8.4 Hz, 2H), 7.19-7.08 (m, 5H), 7.05-7.01 (m, 1H), 6.81 (d, *J* = B.4 Hz, 2H), 6.56 (d, *J* = 8.4 Hz, 2H), 4.55 (s, 2H), 3.72 (s, 3H), 3.60 (t, *J* = 7.6 Hz, 2H), 3.50-3.48 (m, 2H), 3.47-3.36 (m, 2H), 3.21-3.15 (m, 1H), 2.82 (t, *J* = 7.6 Hz, 2H), 2.24 (t, *J* = 2.8 Hz, 2H) |
| 38 | ¹H NMR (400 MHz, MeOD) δ 7.30 (t, *J* = 8.8 Hz, 2H), 7.19-7.1B (m, 1H), 7.17-1.13 (m, 2H), 7.12-7.11 (m, 2H), 7.05-7.01 (m, 1H), 6.81 (d, *J* = B.4 Hz, 2H), 6.56 (d, *J =* 8.4 Hz, 2H), 4.56 (s, 2H), 3.72 (s, 3H), 3.60 (t, *J* = 7.2 Hz, 2H), 3.49-3.48 (m, 2H), 3.39-3.35 (m, 2H), 2.83 (t, *J* = 7.6 Hz, 2H), 2.28-2.24 (m, 2H) |
| 39 | ¹H NMR (400 MHz, DMSO) δ 7.34-7.27 (m, 2H), 7.16 (t, J = 9.2 Hz, 4H), 6.96-6.91 (m, 1H), 6.89-6.85 (m, 2H), 6.43 (d, J = 8.0 Hz, 2H), 4.60 (s, 2H), 3.97 (t, *J* = 8.0 Hz, 2H), 3.62 (t, J = 6.0 Hz, 2H), 3.71 (s, 3H), 3.61 (d, *J* = 7.6 Hz, 2H), 3.48 (d, J = 8.4 Hz, 1H), 2.85 (t, J = 8.0 Hz, 2H) |
| 40 | ¹H NMR (400 MHz, DMSO) δ 7.38 (d J = 8.4 Hz, 2H), 7.33-7.27 (m, 4H), 7.11 (t, J = 6.0 Hz, 1H), 6.53 (d. J = 7.6 Hz, 1H), 6.48 (s, 1H), 6.44 (d. J = 8.4 Hz. 1H), 4.68 (s, 2H), 3.74 (t. J = 7.2 Hz, 2H), 3.24-3.17 (m, 4H), 3.03-2.99 (m, 3H), 2.11 (q, J = 8.4 Hz, 2H) |
| 41 | ¹H NMR (400 MHz, MeOD) δ 7.37-7.20 (m, 2H), 7.22 (t, J = 8.0 Hz, 1H), 6.96-6.95 (m, 2H), 6.87-6.83 (m, 1H), 6.80 (d, J = 8.4 Hz, 2H), 6.74 (d. J = 8.0 Hz, 1H), 6.63-6.58 (m, 2H), 5.50 (q, J = 6.0 Hz, 1H), 3.74 (s, 3H), 3.52-3.38 (m, 6H), 3.16-2.85 (m, 2H), 2.29-2.24 (m, 3H), 1.63 (d, J = 6.8 Hz, 3H) |
| 42 | ¹H NMR (400 MHz, MeOD) δ 7.36-7.26(m, 4H), 6.95 (dd, J = 8.4, 1.6 Hz, 2H), 6.86-6.77 (m, 3H), 6.62 (d, J = 8.8 Hz, 2H), 5.46 (d. J = 6.8 Hz, 1H), 3.74 (s, 3H), 3.53-3.15 (m, 7H), 2.31-2.23 (m, 3H), 1.60 (d, J = 6.8 Hz, 3H) |
| 43 | ¹H NMR (400 MHz, DMSO) δ 12.45 (s, 1H), 7.28-7.21 (m, 1H), 7.17 (t, J = 7.6 Hz, 4H), 6.86 (d, J = 8.4 Hz, 2H), 6.53 (d, J = 8.4 Hz, 2H), 4.61 (s, 2H), 3.71 (s, 3H), 3.42-3.16 (m, 7H), 2.86 (t, J = 7.2 Hz, 2H), 2.17-2.12 (m, 2H) |
| 44 | 1H NMR (400 MHz, DMSO) δ 7.89-7.87 (m, 1H), 7.67-7.64 (m, 1H), 7.61 (d, J = 8.0 Hz. 2H), 7.21 (dd, J = 7.6, 5.2 Hz, 1H), 7.16 (d, J = 8.4 Hz. 2H), 6.84 (d, J = 8.8 Hz, 2H), 4.79 (s, 2H), 3.71 (s, 5H), 2.89 (t, J = 7.2 Hz, 2H) |
| 45 | 1H NMR (400 MHz, DMSO) δ 7.67 (d, J = 8.8 Hz. 1H), 7.59 (d, J = 8.4 Hz, 2H), 7.38 (d. J = 8.0 Hz, 2H), 7.16 (d, J = 8.4 Hz. 2H), 6.85 (d, J = 8.4 Hz, 2H), 6.57 (d, J = 8.8 Hz, 1H), 4.78 (s, 2H), 3.71 (s, 3H), 3.64 (t, J = 7.6 Hz, 2H), 2.87 (t, J = 7.6 Hz, 2H) |
| 46 | 1H NMR (400 MHz, DMSO) δ 12.26 (s, 1H), 7.33 (m 2H), 7.18 (m, 4H), 6.95 (m, 3H), 6.87 (d, J = 8.8 Hz, 2H), 4.63 (s, 2H), 3.71 (s, 3H), 3.61 (m, 4H), 2.86 (t, J = 7.2 Hz, 2H), 2.84 (bs, 2H), 2.50 (m, 1H), 1.88 (d, J = 11.2, 2H), 1.62 (m, 2H) |
| 47 | ¹H NMR (400 MHz, DMSO) δ 12.70 (s, 1H), 9.00 (s, 1H), 8.07 (s, 1H), 7.90 (d, J = 8.8 Hz, 2H), 7.48 (d, J = 8.4 Hz. 2H), 7.32 (m, 2H), 7.20 (d, J = 8.8 Hz. 2H), 6.96 (m, 1H), 6.87 (d, J = 8.4 Hz, 2H), 4.83 (s, 2H), 3.71 (bs, 5H), 2.92 (t, J = 8.0 Hz, 2H) |
| 48 | ¹H NMR (400 MHz, DMSO) δ 7.91 (s, 1H), 7.81 (d, J = 8.4 Hz, 2H), 7.31 (m 5H), 7.16 (d, J = 8.4 Hz, 2H), 6.94 (m, 1H), 6.51 (d, J = 8.4 Hz, 2H), 4.59 (s, 2H), 3.68 (t, J = 7.6 Hz, 2H), 3.22~3.38 (m, 6H), 3.00 (t, J = 7.6 Hz, 2H), 2.15 (m, 2H) |
| 49 | ¹H NMR (400 MHz, DMSO) δ 7.53 (dd, J = 2.0, 7.2 Hz, 1H), 7.34 (m, 1H), 7.27 (m, 2H), 7.35 (d, J = 8.4 Hz, 2H), 6.92 (M, 1H), 6.51 (d, J = 8.4 Hz, 2H), 6.36 (d, J = 9.2 Hz, 1H), 6.12 (td, J = 0.8, J = 6.4 Hz, 1H), 4.58 (s, 2H), 4.17 (t, J = 5.6 Hz, 2H), 3.78 (t, J = 5.6 Hz, 2H), 3.41 (m, 2H), 3.25 (m, 2H), 3.15 (m, 1H), 2.15 (m, 2H) |

### Example 2: Evaluation of Immune Cell Migration Inhibition Activity

The immune cell migration inhibition activity of the 49 synthesized compounds was evaluated.

Cell migration was assessed based on the method described in the prior literature (Park, S. G. et al. Human lysyl-tRNA synthetase is secreted to trigger proinflammatory response. Proceedings of the National Academy of Sciences, 2005, 102.18: 6356-6361.). The specific experimental method is as follows. A Transwell (Corning#3421-5µm pore) was coated with gelatin (0.5mg/ml), and then Raw 264.7 cells (1 x 10⁵ cells/well) were seeded in the upper chamber. Serum-free medium (Serum Free DMEM, 500 µl) containing LN (Laminin 421 1 µg/ml) was added to the lower chamber. Each compound was treated at a concentration of 3µM in the upper chamber. After 24 hours, the cells were fixed with 70% methanol for 30 minutes and stained with 50% hematoxylin for 30 minutes. Non-migrating cells on the upper side of the membrane were removed with a cotton swab, and the membrane was mounted on a slide. Migrating cells on the lower side of the membrane were observed under a high-power microscope, and the number of cells was measured from the obtained images and expressed as the inhibition rate (% inhibition) compared to the control group.

When the control substance C0001 was treated at 3 µM, the % inhibition of cell migration induced by LN was an average of 41.9.

### [Control Substance: C0001]

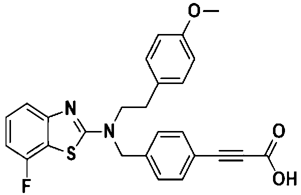

**[Table 3]**

| No. | Migration inhibitor % (3uM) | No. | Migration inhibitor % (3uM) |
|---|---|---|---|
| 1 | 8.8 | 26 | 48 |
| 2 | 15.7 | 27 | 9 |
| 3 | 0 | 28 | 16.4 |
| 4 | 88 | 29 | 33 |
| 5 | 77.7 | 30 | 37.4 |
| 6 | 5.1 | 31 | 72.8 |
| 7 | 10.9 | 32 | 45 |
| 8 | 27 | 33 | 15.7 |
| 9 | 0 | 34 | 13 |
| 10 | 0 | 35 | - |
| 11 | 12 | 36 | 21.8 |
| 12 | 43 | 37 | 19.3 |
| 13 | 90 | 38 | 15.3 |
| 14 | 32 | 39 | 51.9 |
| 15 | 0 | 40 | 37.7 |
| 16 | 33 | 41 | 58.7 |
| 17 | 42 | 42 | 63.1 |
| 18 | 9 | 43 | 31.1 |
| 19 | 15 | 44 | 1.1 |
| 20 | 18 | 45 | 7.8 |
| 21 | 36 | | |
| 22 | 19 | | |
| 23 | 59 | | |
| 24 | 68 | | |
| 25 | 83 | | |

### Example 3: Cell-Free ELISA Analysis to Confirm Inhibition of KARS1 and 37LR Binding

Next, based on the mechanism of action, a cell-free ELISA analysis was conducted to select compounds with high inhibitory activity on the binding of KARS1 and LR (laminin receptor).

The experiment was conducted by treating LRF (TRX-His-Laminin receptor full length) and the compound at 10 µM together on a plate coated with KARS1 (1-207aa) and reacting for 1 hour to confirm whether the compound inhibits the protein binding between KARS1 and 37LR (Table 4).

As a control substance, a KARS1 inhibitor (C0001) synthesized in another study by the inventors was used. When the control substance C0001 was treated at 10 µM, the % inhibition of protein binding between KARS1 and 37LR was 64.5.

**[Table 4]**

| No. | KARS1-37LR (% inhibition, 10uM) | No. | KARS1-37LR (% inhibition, 10uM) |
|---|---|---|---|
| C0001 | 64.5 | 26 | 78.4 |
| 1 | -6.0 | 27 | 14.0 |
| 2 | 79.4 | 28 | - |
| 3 | 4.7 | 29 | - |
| 4 | 13.8 | 30 | 64.0 |
| 5 | 0.8 | 31 | - |
| 6 | 14.8 | 32 | -27.9 |
| 7 | 9.5 | 33 | 41.9 |
| 8 | 13.4 | 34 | - |
| 9 | 8.5 | 35 | - |
| 10 | 9.8 | 36 | - |
| 11 | 3.0 | 37 | - |
| 12 | 57.9 | 38 | - |
| 13 | 83.9 | 39 | 37.5 |
| 14 | 100.0 | 40 | 66.7 |
| 15 | 86.4 | 41 | 69.6 |
| 16 | 68.2 | 42 | 65.6 |
| 17 | 79.0 | 43 | 67.1 |
| 18 | 86.2 | 44 | - |
| 19 | 75.8 | 45 | - |
| 20 | 3.4 | | |
| 21 | 8.9 | | |
| 22 | 52.0 | | |
| 23 | 71.6 | | |
| 24 | 65.9 | | |
| 25 | 69.2 | | |

### Example 4: Inhibition of KARS1-Dependent Cell Migration

C0001 inhibits the binding of KARS1 and 67LR, thereby blocking the migration of monocytes/macrophages and suppressing inflammation. Accordingly, a trans-migration assay using mouse macrophages (Raw264.7) was conducted to preliminarily screen drugs that inhibit the migration of monocytes/macrophages.

It is known that KARS1 is secreted extracellularly in an inflammatory environment, inducing the migration and M1 polarization of macrophages and peripheral blood monocytes.

Based on this, when KARS1 protein was directly treated externally instead of LN, the trans-migration of Raw264.7 was induced, and the cell migration inhibitory effect of the KARS1 inhibitor C0001 was confirmed. It was verified that C0001, which specifically binds to KARS1, can inhibit cell migration by binding to extracellular KARS1, in addition to the mechanism of inhibiting cell membrane migration of KARS1 induced by laminin and its binding to 67LR, thereby enabling this analysis to be used as a direct method for evaluating *in vitro* activity against KARS1.

An analysis method to directly inhibit cell migration induced by KARS1 was established, and preliminary candidate substances were treated at a concentration of 1 µM for analysis (Table 5).

As a control substance, a KARS1 inhibitor (C0001) synthesized in another study by the inventors was used. When the control substance C0001 was treated at 1 µM, the % inhibition of cell migration induced by KARS1 was 68.7.

**[Table 5]**

| No. | KARS1-induced Raw264.7 cell migration (% inhibition, 1uM) | No. | KARS1-induced Raw264.7 cell migration (% inhibition, 1uM) |
|---|---|---|---|
| C0001 | 68.7 | 26 | 80.6 |
| 1 | 19.8 | 27 | 9.1 |
| 2 | 98.1 | 28 | 43.2 |
| 3 | 22.6 | 29 | 4.1 |
| 4 | 9.8 | 30 | 3.2 |
| 5 | 95.6 | 31 | 28.3 |
| 6 | 23.5 | 32 | 18.1 |
| 7 | 39.1 | 33 | 57.6 |
| 8 | - | 34 | 99.4 |
| 9 | - | 35 | - |
| 10 | 11.7 | 36 | 96.8 |
| 11 | 37.8 | 37 | 50.6 |
| 12 | 55.1 | 38 | 40.4 |
| 13 | 93.4 | 39 | 97.4 |
| 14 | 73.4 | 40 | 76.1 |
| 15 | 54.4 | 41 | 90.2 |
| 16 | 86.2 | 42 | 85.8 |
| 17 | 5.6 | 43 | 98.4 |
| 18 | 31 | 44 | - |
| 19 | 69 | 45 | 39.8 |
| 20 | 49.1 | 46 | 90.9 |
| 21 | 26.1 | 47 | 99.7 |
| 22 | 43.4 | 48 | 36.6 |
| 23 | 35.4 | 49 | 34.8 |
| 24 | 96.3 | | |
| 25 | 96.2 | | |

Next, preliminary candidate substances with high inhibitory activity at 1 µM were treated at concentrations of 0.1~4 µM for analysis.

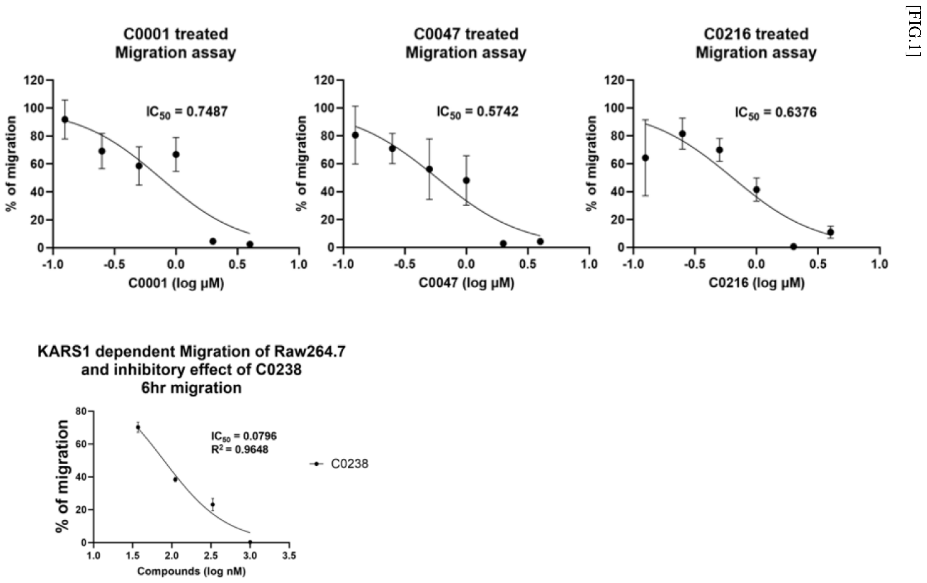

As a result, EC50 values of 0.75, 0.57, and 0.64 µM were obtained for C0001, Compound 25 (C0047), and Compound 43 (C0216), respectively, which showed similar activity patterns to the EC50 values of 7.93, 5.96, and 5.09 µM for cell migration inhibition induced by LN but exhibited approximately 10 times higher sensitivity. The compound with the highest activity was Compound 47 (C0238), with an EC50 of 0.08 µM, showing approximately 10 times higher activity compared to C0001 (Figure 1).

### Example 5: Evaluation of Cancer Cell Migration Inhibition Activity

The cancer cell migration inhibition activity of three compounds (C0001, Compound 25 (C0047), Compound 43 (C0216)) was evaluated.

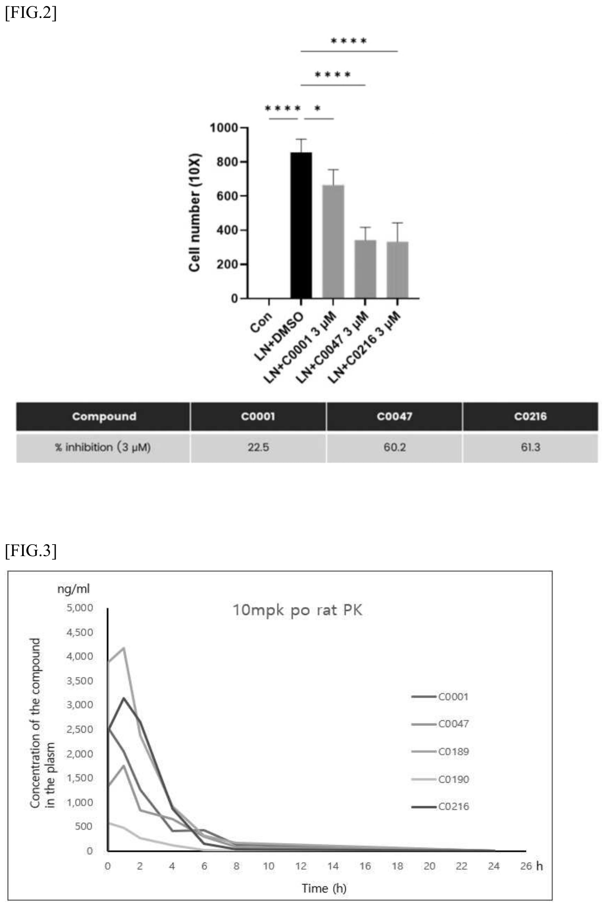

The specific experimental method for cell migration is as follows. The upper chamber of a Transwell (Corning#3422-8µm pore) was inverted, and 70 µL of LN (Laminin 421 5 µg/mL) diluted in DPBS was dispensed onto the upper surface and coated at 4°C for 24 hours. Subsequently, A549 cells (5 x 104 cells/well) were seeded inside the upper chamber, and serum-free medium (Serum Free RPMI 700 µL) was added to the lower chamber. Each compound was treated at a concentration of 3 µM in the lower chamber, and for the drug control group, DMSO was treated at the same ratio as the compound. The cells were incubated for 6 hours in a 5% CO₂ incubator. The cells were fixed with 70% ethanol for 1 hour and stained with 0.1% crystal violet for 20 minutes. Non-migrating cells on the upper side of the membrane were removed with a cotton swab, and the membrane was dried at room temperature for 24 hours. Migrating cells on the lower side of the membrane were observed under a high-power microscope at 100X magnification, and the number of cells was measured from the obtained images using the Image-J program (Figure 2).

### Example 6: Evaluation of Metabolic Stability

Metabolic stability (MS) evaluation was conducted for the selected compounds by measuring *in vitro* stability in two species, rat and human, according to the microsomal stability measurement method. As a result, it was identified that MS varied depending on the transformation of parts A, B, and C.

The MS results, confirmed as remaining % over 30 minutes for rat and human microsomal stability, are shown in Table 6 for six compounds that showed improvement compared to C0001.

**[Table 6]**

| No. | Metabolic stability (% remaining at 30 min) | |
|---|---|---|
| | Rat | Human |
| C0001 | 50 | 56 |
| 13(C0030) | 90 | 74 |
| 17(C0034) | 71 | 58 |
| 25(C0047) | 77 | 66 |
| 41(C0189) | 58 | 51 |
| 42(C0190) | 43 | 76 |
| 43(C0216) | 80 | 85 |

Among these, secondary MS measurements were conducted for Compound 25 (C0047), Compound 43 (C0216), and C0001 using the S9 method. This test measured the remaining % at 30, 45, and 60 minutes for four species: mouse, rat, dog, and human. As a result, Compounds 25 and 43 showed improved S9 stability in all four species compared to C0001, with Compound 43 exhibiting the most stable metabolic stability (Table 7).

In particular, the compounds not only showed over 70% stability in human MS up to 60 minutes but also exhibited similar stability across all four species (mouse, rat, dog, human), suggesting that it could enable predictable and stable evaluations in efficacy and toxicity tests during future drug development.

**[Table 7]**

| No. | Metabolic stability (% remaining at each time) | | | | | | | | | | | |
|---|---|---|---|---|---|---|---|---|---|---|---|---|
| | Mouse (min) | | | Rat (min) | | | Dog (min) | | | Human (min) | | |
| | 30 | 45 | 60 | 30 | 45 | 60 | 30 | 45 | 60 | 30 | 45 | 60 |
| C0001 | 67.1 | 62.3 | 49.6 | 79.6 | 76.6 | 83.1 | 80.7 | 85.6 | 75.9 | 59.1 | 50.2 | 46.2 |
| 25 (C0047) | 70.5 | 71.7 | 60.9 | 86.0 | 84.1 | 88.5 | 100.6 | 93.5 | 97.0 | 70.1 | 75.8 | 66.8 |
| 43 (C0216) | 79.8 | 77.7 | 73.2 | 99.2 | 81.6 | 88.0 | 110.9 | 117.3 | 100.6 | 87.3 | 80.7 | 92.4 |

### Example 8: Pharmacokinetics Analysis

Pharmacokinetics (PK) analysis was conducted in rats for five compounds that showed primary improvement in MS compared to C0001 in rat and human. For this purpose, formulations suitable for peroral administration (po) and intravenous injection (iv) for each compound were first selected to ensure stable PK progression.

The formulation was selected under the condition of maintaining a clear solution for more than 1 hour at 10 and 30 mpk doses based on po, and the iv formulation was modified when iv use was not possible based on po (Table 8).

**[Table 8]**

| No. | Dose (mpk) | Administration | formulation |
|---|---|---|---|
| C0001 | 2 | iv | 50% PEG400 + 50% water |
| | 10 | po | 40% PEG400 + 60% (7.6% poloxamer solution) |
| | 30 | po | 40% PEG400 + 60% (7.6% poloxamer solution) |
| 25 (C0047) | 2 | iv | 40% PEG400 + 60% (7.6% poloxamer solution) |
| | 10 | po | 40% PEG400 + 60% (7.6% poloxamer solution) |
| | 30 | po | 5% DMSO + 5% Cremophor or EL + 90% water |
| 41 (C0189) | 10 | po | 40% PEG400 + 60% (7.6% poloxamer solution) |
| | 30 | po | 40% PEG400 + 60% (7.6% poloxamer solution) |
| 42 (C0190) | 10 | po | 40% PEG400 + 60% (7.6% poloxamer solution) |
| 43 (C0216) | 2 | iv | 40% PEG400 + 60% (7.6% poloxamer solution) |
| | 10 | po | 5% DMSO + 5% Tween80 + 40% PEG400 + 50% water |
| | 30 | po | 5% DMSO + 20% PEG400 + 5% solutol + 70% water |

To confirm the oral administration potential of the selected compounds, 10 mpk rat po PK was conducted. The PK profile and key indicators after administering 10 mpk to five compounds, C0001, 25, 41, 42, and 43, are shown in Figure 3 and Table 9.

Among the five compounds, 42 showed the lowest blood exposure with an AUC value of 1,425 ng*h/ml, while C0001, 25, 41, and 43 exhibited AUC values exceeding 5,000 ng*h/ml, confirming the potential for oral administration. In particular, compound 41, which had the highest AUC value, showed a pharmacokinetic profile with an AUC value of 11,859 µg*h/ml in the 10 mpk po PK, which was more than twice that of C0001.

**[Table 9]**

| Compound ID | No. of animals | T1/2 (h) | Tmax (h) | Cmax (ng/ml) | AUC (ng*h/ml) |
|---|---|---|---|---|---|
| C0001 | 3 | 1.83 | 0.5 | 2,015 | 5,828 |
| 25 (C0047) | 3 | 1.34 | 1.0 | 1,754 | 5,109 |
| 41 (C0189) | 3 | 1.96 | 0.83 | 4,232 | 11,859 |
| 25 (C0047) | 3 | 1.32 | 0.67 | 240 | 1,425 |
| 43 (C0216) | 3 | 0.95 | 1.33 | 3,324 | 9,711 |

Although no substance showed an improved half-life compared to C0001, compounds 25, 41, and 43 exhibited different patterns in Tmax and pharmacokinetic profiles compared to C0001. Notably, compounds 25 and 43 showed different drug elimination patterns in the blood, and considering the improved stability of these two substances in the previous MS, it was possible to predict differentiated *in vivo* profile patterns compared to C0001.

Based on the previous 10 mpk po rat PK results, 30 mpk po rat PK was conducted for four compounds, C0001, 25, 41, and 43, to confirm changes in the blood PK profile of the substances with increasing doses.

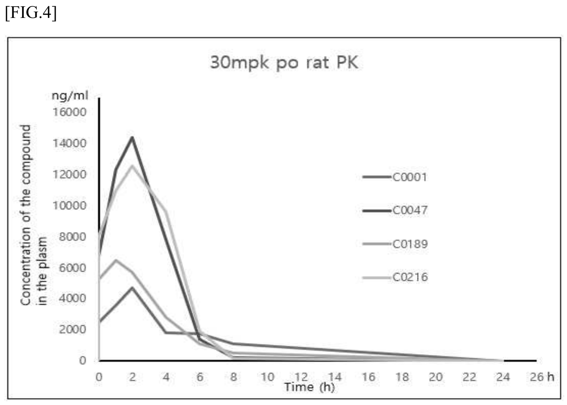

As a result, compounds 25, 41, and 43 all showed higher exposure than C0001. In the case of compound 41, which showed the highest exposure in the 10 mpk po PK, the AUC value increased approximately 2.4 times at 30 mpk, but it did not show a higher AUC increase rate than C0001. On the other hand, compounds 25 and 43 showed dose-dependent increases in exposure, with increases of 10.4 times and 5.7 times, respectively (Figure 4 and Table 10).

**[Table 10]**

| Compound ID | No. of animals | T1/2 (h) | Tmax (h) | Cmax (ng/ml) | AUC (ng*h/ml) |
|---|---|---|---|---|---|
| C0001 | 3 | 2.67 | 2.0 | 4,717 | 25,602 |
| 25 (C0047) | 3 | 0.76 | 2.0 | 14,375 | 52,969 |
| 41 (C0189) | 3 | 2.13 | 1.0 | 6,469 | 28,533 |
| 43 (C0216) | 3 | 1.00 | 1.67 | 12,760 | 55,279 |

### Example 9: Measurement of Bioavailability (BA)

Through the 10 and 30 mpk po rat PK, two compounds, 25 and 43, which showed higher blood exposure than C0001 and dose-dependent increases in exposure, were secured. These two compounds were rapidly eliminated in the blood, had lower half-lives compared to C0001, and were expected to show differentiated *in vivo* profiles, such as differences in organ distribution patterns. In the case of compound 41, although it showed high blood exposure at low doses, dose-dependent increases in exposure were not observed, and it showed a similar half-life in blood elimination rates, suggesting that it would not exhibit a differentiated *in vivo* profile.

Therefore, although C0001 and the target binding inhibitory activity were similar, compounds 25 and 43, which showed slightly improved *in vitro* cell migration inhibitory efficacy and were expected to have differentiated *in vivo* profiles, were selected as the final preliminary candidates along with one other compound. For these compounds, 2 mpk iv rat PK was conducted to calculate bioavailability (BA) (Table 11).

**[Table 11]**

| Compound ID | Route of administration (No. of animals) | Dose (mpk) | AUC (ng·h/ml) | BA (%) |
|---|---|---|---|---|
| C0001 | iv (3) | 2 | 3,436 | - |
| | po (3) | 10 | 5,828 | 33.9 |
| | po (3) | 30 | 25,602 | 49.6 |
| 25 (C0047) | iv (3) | 2 | 2,408 | - |
| | po (3) | 10 | 5,409 | 44.9 |
| | po (3) | 30 | 52,969 | 146.6 |
| 43 | iv (3) | 2 | 2,689 | - |
| (C0216) | po (3) | 10 | 9,711 | 72.2 |
| | po (3) | 30 | 55,279 | 137.0 |

The preliminary candidates, compounds 25 and 43, showed higher BA compared to C0001, and dose-dependency was also confirmed. Therefore, compounds 25 and 43 were expected to have high drugability as orally administered drugs.

### Example 10: Measurement of Organ Distribution

Although C0001 and the target binding inhibitory activity were similar, compounds 25 and 43, which showed slightly improved *in vitro* cell migration inhibitory efficacy and were expected to have differentiated *in vivo* profiles, including bioavailability, were selected as the primary preliminary candidates, and the distribution patterns in various organs were analyzed.

Preliminary candidate compound 25 showed a pattern of increased plasma exposure within a short time compared to C0001, followed by a rapid decrease, while compound 43 also showed increased plasma exposure within a short time but exhibited a somewhat slower decrease pattern. Furthermore, unlike C0001, which showed a liver-specific distribution pattern, the two selected compounds showed high distribution across various organs. Therefore, compounds 25 and 43 not only exhibited high systemic exposure and drugability as orally administered drugs but also showed distribution patterns across various organs, indicating the potential for development as differentiated drugs compared to C0001 (Table 12).

**[Table 12]**

| Compound ID | Organ distribution (ratio per plasma at 0.5 / 6.0 h) | | | | | |
|---|---|---|---|---|---|---|
| Organ | Plasma (ng/ml) | Brain | Lung | Heart | Kidney | Liver |
| C0001 | 3,145 / 1,769 | 0.02 / 0.01 | 0.08 / 0.06 | 0.06 / 0.03 | 0.01 / 0.01 | 19.1 / 19.1 |
| 25 (C0047) | 29,901 / 1,499 | 0.06 / 0.54 | 0.41 / 1.05 | 0.29 / 0.52 | 0.38 / 0.79 | 0.85 / 3.15 |
| 43 (C0216) | 19,420 / 5,366 | 0.09 / 0.92 | 4.30 / 0.55 | 0.42 / 0.35 | 0.61 / 1.89 | 1.70 / 3.56 |

### [Industrial Applicability]

The novel compounds of the present invention can regulate the migration of immune cells and cancer cells by inhibiting the activity of KARS1, and thus exhibit very remarkable effects in the prevention, improvement, and treatment of diseases related to immune cell migration and cancer metastasis, thereby having very high industrial applicability.

## Claims

1. A compound of Formula 1, an isomer thereof, or a pharmaceutically acceptable salt thereof: 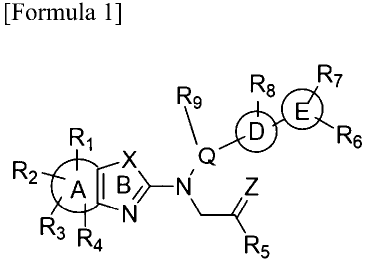 wherein
X is O, NH, S or CH=N,
A is C5-C10 aryl forming a bicyclic aromatic ring or 5- to 10-membered heteroaryl containing one or more heteroatoms selected from the group consisting of N, O and S, together with B
R₁, R₂, R₃, R₄ are each independently absent, hydrogen, halogen, =O, C1-C6 alkyl or amine,
Z is two H or O,
R₅ is C5-C10 aryl or pyridinonyl substituted or unsubstituted with C1-C6 alkoxy, -(C=O)NH₂ or - OCF₃,
Q is hydrogen or methylene,
D is carboxyl, C5-C10 aryl, benzoimidazolyl, benzoxazolyl, isoindolinyl, -C=O-, -C₆H₄C=O- or - C₆H₄NH-,
R₈ is hydrogen or C1-C6 alkyl,
E is, when D is aryl, phenyl, C1-C6 alkylene, C2-C8 alkylaminocarbonyl, 3- to 10-membered heterocycloalkyl containing one or more heteroatoms selected from the group consisting of N, O and S, 5- to 10-membered heteroaryl containing one or more heteroatoms selected from the group consisting of N, O and S, C3-C10 cycloalkylamine, C3-C10 cycloalkyl, -B(OH)O-, - CH₂CH₂(C=O), -C=C-, -C=C-(C=O)N-, -cyclobutyl-(C=O)-, -azetinyl-(C=O)-, -C₆H₄-(C=O)-, - pyrrolidinyl-(C=O)-, -furanyl-(C=O)-, -piperidinyl-(C=O)-, -pyrazolyl-(C=O)-, or pyrrolyl-(C=O)-, wherein R₆, R₇ are each independently absent, hydrogen, carboxyl, C1-C6 alkyl, C2-C8 alkylaminocarbonyl, aminocarbonyl, hydroxy or -Si(CH₃)₃,
E is, when A is pyrrole or pyridone and D is aryl, hydrogen, halogen, -C=C-, -C=C-(C=O), wherein R₆, R₇ are each independently absent, hydrogen, carboxyl, OH, CH₃, CH₂CH₃, -Si(CH₃)₃,
E is, when D is -C=O-, benzoimidazolyl, benzoxazolyl, isoindolinyl, -C₆H₄C=O- or -C₆H₄NH-, absent, -B(OH)₂, carboxyl, hydrogen, aminocarbonyl, aminosulfonyl, C1-C6 alkylene, -(C=O)-, - C=C-, -S(=O)₂-, -CH₂(C=O)-, -B(OH)O-, -CHR₁₀(C=O)-, -NHCHR₁₀(C=O)-, cyclobutyl-(C=O)-, furanyl-(C=O)- or pyrrolidinyl-(C=O)-, wherein R₆, R₇ are each independently absent, carboxyl, hydrogen, hydroxy, C1-C6 alkyl or amine,
R₉ is hydrogen or C1-C6 alkyl,
R₁₀ is hydrogen, C1-C6 alkyl or C1-C6 hydroxyalkyl.

2. The compound, isomer thereof, or pharmaceutically acceptable salt thereof according to claim 1, wherein A is benzene, pyridine, pyrimidine, pyridone or pyrrole forming a bicyclic aromatic ring together with B.

3. The compound, isomer thereof, or pharmaceutically acceptable salt thereof according to claim 1, wherein the bicyclic aromatic ring formed by A and B is selected from the following structures:

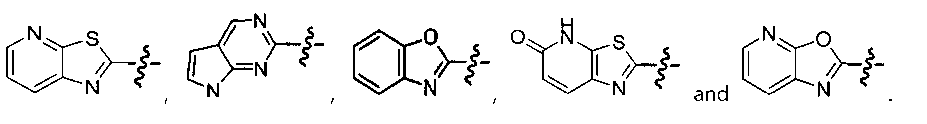

4. The compound, isomer thereof, or pharmaceutically acceptable salt thereof according to claim 1, wherein R₅ is phenyl substituted with methoxy, -(C=O)NH₂ or -OCF₃, or pyridinonyl.

5. The compound, isomer thereof, or pharmaceutically acceptable salt thereof according to claim 1, wherein D is phenyl, carboxyl, benzoimidazolyl, benzoxazolyl or isoindolinyl.

6. The compound, isomer thereof, or pharmaceutically acceptable salt thereof according to claim 1, wherein when D is aryl and A is not pyrrole or pyridone, E is phenyl, ethylene, -C=C-, pyrrolidinyl, pyrrolidinylcarbonyl, methylaminocarbonyl, furanyl, cyclobutylamino, pyrrolyl, cyclobutyl or azetidinyl, wherein R₆, R₇ are each independently absent, hydrogen, carboxyl, diethylaminocarbonyl, methylaminocarbonyl, aminocarbonyl, -Si(CH₃)₃ or isopropyl.

7. The compound, isomer thereof, or pharmaceutically acceptable salt thereof according to claim 1, wherein when D is aryl and A is pyrrole or pyridone, E is halo or -C=C-, wherein R₆, R₇ are each independently absent, hydrogen, Si(CH₃)₃ or carboxyl.

8. The compound, isomer thereof, or pharmaceutically acceptable salt thereof according to claim 1, wherein when D is benzoimidazolyl, benzoxazolyl or isoindolinyl, E is hydrogen, -B(OH)₂, carboxyl, aminocarbonyl, aminosulfonyl or methylene, wherein R₆, R₇ are each independently absent or carboxyl.

9. The compound, isomer thereof, or pharmaceutically acceptable salt thereof according to claim 1, wherein the compound of Formula 1 is selected from the group consisting of:
N-([1,1'-biphenyl]-4-ylmethyl)-N-(4-methoxyphenethyl)thiazolo[5,4-b]pyridin-2-amine, 3-(4-(((7-fluorobenzo[d]thiazol-2-yl)(4-methoxyphenethyl)amino)-methyl)phenyl)propanoic acid,
N,N-diethyl-3-(4-(((7-fluorobenzo[d]thiazol-2-yl)(4-methoxyphen-ethyl)amino)methyl)phenyl)propiolamide,
3-(4-(((7-fluorobenzo[d]thiazol-2-yl)(4-methoxyphenethyl)amino)-methyl)phenyl)-N-methylpropiolamide,
3-(4-(((7-fluorobenzo[d]thiazol-2-yl)(4-methoxyphenethyl)amino)-methyl)phenyl)propiolamide,
N-(4-methoxyphenethyl)-7-methyl-7H-pyrrolo[2,3-d]pyrimidin-2-amine,
N-(4-bromobenzyl)-N-(4-methoxyphenethyl)-7-methyl-7H-pyrrolo[2,3-d]pyrimidin-2-amine,
7-fluoro-N-(4-methoxyphenethyl)-N-(3-((trimethylsilyl)ethynyl)-benzyl)benzo[d]thiazol-2-amine,
N-(3-ethynylbenzyl)-7-fluoro-N-(4-methoxyphenethyl)benzo[d]thiazol-2-amine,
N-(4-methoxyphenethyl)-7-methyl-N-(4-((trimethylsilyl)ethynyl)-benzyl)-7H-pyrrolo[2,3-d]pyrimidin-2-amine,
N-(4-ethynylbenzyl)-N-(4-methoxyphenethyl)-7-methyl-7H-pyrrolo[2,3-d]pyrimidin-2-amine, 3-(4-(((4-methoxyphenethyl)(7-methyl-7H-pyrrolo[2,3-d]pyrimidin-2-yl)amino)methyl)phenyl)propiolic acid,
1-(4-(((7-fluorobenzo[d]thiazol-2-yl)(4-methoxyphenethyl)amino)-methyl)phenyl)pyrrolidine-3-carboxylic acid,
(4-(((7-fluorobenzo[d]thiazol-2-yl)(4-methoxyphenethyl)amino)-methyl)benzoyl)-L-proline,
1-(4-(((7-fluorobenzo [d]thiazol-2-yl)(4-methoxyphenethyl)amino)-methyl)benzoyl)pyrrolidine-3-carboxylic acid,
(4-(((7-fluorobenzo[d]thiazol-2-yl)(4-methoxyphenethyl)amino)-methyl)benzoyl)glycine,
1-(3-(((7-fluorobenzo[d]thiazol-2-yl)(4-methoxyphenethyl)amino)-methyl)phenyl)pyrrolidine-3-carboxylic acid,
(4-(((7-fluorobenzo[d]thiazol-2-yl)(4-methoxyphenethyl)amino)-methyl)benzoyl)-D-valine,
(4-(((7-fluorobenzo[d]thiazol-2-yl)(4-methoxyphenethyl)amino)-methyl)phenyl)-L-proline,
(3-(((7-fluorobenzo[d]thiazol-2-yl)(4-methoxyphenethyl)amino)-methyl)phenyl)-L-proline,
(2-(((7-fluorobenzo[d]thiazol-2-yl)(4-methoxyphenethyl)amino)- methyl)-1-methyl-1H-benzo[d]imidazol-6-yl)boronic acid,
(S)-1-(3-(((7-fluorobenzo[d]thiazol-2-yl)(4-methoxyphenethyl)amino)-methyl)phenyl)pyrrolidine-3-carboxylic acid,
(R)-1-(3-(((7-fluorobenzo[d]thiazol-2-yl)(4-methoxyphenethyl)amino)-methyl)phenyl)pyrrolidine-3-carboxylic acid,
(R)-1-(4-(((7-fluorobenzo[d]thiazol-2-yl)(4-methoxyphenethyl)-amino)methyl)phenyl)pyrrolidine-3-carboxylic acid,
(S)-1-(4-(((7-fluorobenzo[d]thiazol-2-yl)(4-methoxyphenethyl)amino)-methyl)phenyl)pyrrolidine-3-carboxylic acid,
5-(4-(((7-fluorobenzo[d]thiazol-2-yl)(4-methoxyphenethyl)amino)-methyl)phenyl)furan-2-carboxylic acid,
2-(((7-fluorobenzo[d]thiazol-2-yl)(4-methoxyphenethyl)amino)methyl)-1-methyl-1H-benzo[d]imidazole-6-carboxylic acid,
2-(((7-fluorobenzo[d]thiazol-2-yl)(4-methoxyphenethyl)amino)-methyl)benzo[d]oxazole-5-carboxylic acid,
7-fluoro-N-(isoindolin-5-ylmethyl)-N-(4-methoxyphenethyl)benzo-[d]thiazol-2-amine,
5-(((7-fluorobenzo[d]thiazol-2-yl)(4-methoxyphenethyl)amino)- methyl)isoindoline-2-carboxamide,
5-(((7-fluorobenzo[d]thiazol-2-yl)(4-methoxyphenethyl)amino)-methyl)isoindoline-2-sulfonamide,
2-(5-(((7-fluorobenzo[d]thiazol-2-yl)(4-methoxyphenethyl)amino)-methyl)isoindolin-2-yl)acetic acid,
1-((4-(((7-fluorobenzo[d]thiazol-2-yl)(4-methoxyphenethyl)amino)-methyl)phenyl)amino)cyclobutane-1-carboxylic acid,
1-(4-(((7-fluorobenzo[d]thiazol-2-yl)(4-methoxyphenethyl)amino)-methyl)phenyl)-1H-pyrrole-3-carboxylic acid,
N-(7-fluorobenzo[d]thiazol-2-yl)-N-(4-methoxyphenethyl)glycine,
1-(4-(((7-fluorobenzo[d]thiazol-2-yl)(4-methoxyphenethyl)amino)-methyl)phenyl)cyclobutane-1-carboxylic acid,
(R)-1-(4-((benzo[d]oxazol-2-yl(4-methoxyphenethyl)amino)methyl)-phenyl)pyrrolidine-3-carboxylic acid,
(S)-1-(4-((benzo[d]oxazol-2-yl(4-methoxyphenethyl)amino)methyl)-phenyl)pyrrolidine-3-carboxylic acid,
1-(4-(((7-fluorobenzo[d]thiazol-2-yl)(4-methoxyphenethyl)amino)-methyl)phenyl)azetidine-3-carboxylic acid,
(R)-1-(3-(((7-fluorobenzo[d]thiazol-2-yl)(4-(trifluoromethoxy)-phenethyl)amino)methyl)phenyl)pyrrolidine-3-carboxylic acid,
(3R)-1-(3-(1-((7-fluorobenzo[d]thiazol-2-yl)(4-methoxyphenethyl)-amino)ethyl)phenyl)pyrrolidine-3-carboxylic acid,
(3S)-1-(4-(1-((7-fluorobenzo[d]thiazol-2-yl)(4-methoxyphenethyl)-amino)ethyl)phenyl)pyrrolidine-3-carboxylic acid,
(S)-1-(4-(((4-methoxyphenethyl)(4,5,7-trifluorobenzo[d]thiazol-2-yl)amino)methyl)phenyl)pyrrolidine-3-carboxylic acid,
3-(4-(((4-methoxyphenethyl)(oxazolo[5,4-b]pyridin-2-yl)amino)-methyl)phenyl)propiolic acid,
3-(4-(((4-methoxyphenethyl)(5-oxo-4,5-dihydrothiazolo[5,4-b]pyridin-2-yl)amino)methyl)phenyl)propiolic acid,
1-(4-(((7-fluorobenzo[d]thiazol-2-yl)(4-methoxyphenethyl)amino)-methyl)phenyl)piperidine-4-carboxylic acid,
1-(4-(((7-fluorobenzo[d]thiazol-2-yl)(4-methoxyphenethyl)amino)-methyl)phenyl)-1H-pyrazole-4-carboxylic acid,
(S)-1-(4-(((4-carbamoylphenethyl)(7-fluorobenzo[d]thiazol-2-yl)amino)methyl)phenyl)pyrrolidine-3-carboxylic acid, and
(S)-1-(4-(((7-fluorobenzo[d]thiazol-2-yl)(2-(2-oxopyridin-1(2H)-yl)ethyl)amino)methyl)phenyl)pyrrolidine-3-carboxylic acid.

10. A pharmaceutical composition for preventing or treating immune cell migration-related diseases comprising the compound according to any one of claims 1 to 9, an isomer thereof, or a pharmaceutically acceptable salt thereof as an active ingredient.

11. The composition according to claim 10, wherein the immune cell migration-related disease is selected from the group consisting of cardiovascular disease, fibrotic disease, inflammatory disease and Alport syndrome.

12. The composition according to claim 11, wherein the cardiovascular disease is selected from the group consisting of hypertension, pulmonary arterial hypertension, atherosclerosis, angina pectoris, myocardial infarction, ischemic cerebrovascular disease, arteriolosclerosis and medial sclerosis.

13. The composition according to claim 11, wherein the fibrotic disease is selected from the group consisting of scleroderma, rheumatoid arthritis, Crohn's disease, ulcerative colitis, myelofibrosis, pulmonary fibrosis, hepatic fibrosis, liver cirrhosis, kidney fibrosis, glomerulosclerosis, myofibrosis, cardiac fibrosis, interstitial fibrosis, pancreatic fibrosis, splenic fibrosis, mediastinal fibrosis, vascular fibrosis, skin fibrosis, ocular fibrosis, macular degeneration, joint fibrosis, thyroid fibrosis, endomyocardial fibrosis, peritoneal fibrosis, retroperitoneal fibrosis, progressive massive fibrosis, nephrogenic systemic fibrosis, systemic lupus erythematosus, hereditary fibrosis, infectious fibrosis, irritant fibrosis, fibrosis due to chronic autoimmunity, fibrosis due to antigen incompatibility in organ transplantation, fibrous complications of surgery, fibrosis due to hyperlipidemia, fibrosis due to obesity, diabetic fibrosis, fibrosis due to hypertension and occlusion due to fibrosis during stent insertion.

14. The composition according to claim 11, wherein the inflammatory disease is selected from the group consisting of autoimmune disease, inflammatory bowel disease, dermatitis, atopic dermatitis, eczema, psoriasis, diabetic eye disease, diabetic retinopathy, peritonitis, osteomyelitis, cellulitis, meningitis, encephalitis, pancreatitis, trauma-induced shock, bronchial asthma, rhinitis, sinusitis, otitis media, pneumonia, gastritis, enteritis, cystic fibrosis, stroke, bronchitis, bronchiolitis, hepatitis, cirrhosis, non-alcoholic steatohepatitis, nephritis, proteinuria, diabetic renal failure, arthritis, psoriatic arthritis, neuritis, diabetic neuropathy, multiple sclerosis, gout, spondylitis, Reiter's syndrome, polyarteritis nodosa, vasculitis, amyotrophic lateral sclerosis, Wegener's granulomatosis, hypercytokinemia, polymyalgia rheumatica, giant cell arteritis, calcium crystal deposition arthropathy, pseudogout, non-articular rheumatism, bursitis, tendinitis, epicondylitis, Charcot's joint, hemarthrosis, Henoch-Schönlein purpura, hypertrophic osteoarthropathy, multicentric reticulohistiocytosis, surcoilosis, hemochromatosis, sickle cell disease, hyperlipoproteinemia, hypogammaglobulinemia, hyperparathyroidism, acromegaly, familial Mediterranean fever, Behcet's disease, systemic lupus erythematosus, relapsing fever, psoriasis, multiple sclerosis, sepsis, septic shock, acute respiratory distress syndrome, multiple organ failure, chronic obstructive pulmonary disease, acute lung injury and broncho-pulmonary dysplasia.

15. The composition according to claim 14, wherein the autoimmune disease is selected from the group consisting of rheumatoid arthritis, systemic scleroderma, systemic lupus erythematosus, psoriasis, asthma, ulcerative colitis, Behcet's disease, Crohn's disease, multiple sclerosis, dermatomyositis, collagen disease, vasculitis, arthritis, granulomatosis, organ-specific autoimmune lesions, ulcerative colitis and graft-versus-host disease.

16. A pharmaceutical composition for preventing or inhibiting cancer metastasis comprising the compound, an isomer thereof, or a pharmaceutically acceptable salt thereof according to any one of claims 1 to 9 as an active ingredient.

17. Use of the compound, an isomer thereof, or a pharmaceutically acceptable salt thereof according to any one of claims 1 to 9 for manufacturing a composition for treating immune cell migration-related diseases.

18. A method for treating immune cell migration-related diseases comprising administering an effective amount of a composition comprising the compound, an isomer thereof, or a pharmaceutically acceptable salt thereof according to any one of claims 1 to 9 as an active ingredient to a subject in need thereof.

19. Use of the compound, an isomer thereof, or a pharmaceutically acceptable salt thereof according to any one of claims 1 to 9 for manufacturing a composition for inhibiting cancer metastasis.

20. A method for inhibiting cancer comprising administering an effective amount of a composition comprising the compound, an isomer thereof, or a pharmaceutically acceptable salt thereof according to any one of claims 1 to 9 as an active ingredient to a subject in need thereof.
